(19) Europäisches Patentamt
European Patent Office
Office européen des brevets

(11) **EP 2 054 515 B1**

(12) **EUROPEAN PATENT SPECIFICATION**

(45) Date of publication and mention
of the grant of the patent:
**11.01.2012 Bulletin 2012/02**

(51) Int Cl.:
*C12N 15/10* (2006.01)    *A61K 48/00* (2006.01)
*C12N 7/04* (2006.01)    *A61K 35/76* (2006.01)
*A61P 25/28* (2006.01)

(21) Application number: **07799710.4**

(22) Date of filing: **19.07.2007**

(86) International application number:
**PCT/US2007/073858**

(87) International publication number:
**WO 2008/011503 (24.01.2008 Gazette 2008/04)**

(54) **USE OF A PHAGE DISPLAYING A PEPTIDE FROM AN ADHESION PROTEIN FOR TREATING A DISEASE ASSOCIATED WITH INTRACELLULAR FORMATION OF PROTEIN FIBRILLAR INCLUSIONS OR AGGREGATES**

VERWENDUNG EINES PHAGEN MIT ANZEIGE EINES PEPTIDS EINES ADHÄSIONSPROTEINS ZUR BEHANDLUNG EINER ERKRANKUNG IM ZUSAMMENHANG MIT INTRAZELLULÄRER BILDUNG VON FIBRILLÄREN PROTEINEINSCHLÜSSEN ODER AGGREGRATEN

PROCÉDÉ D'INHIBITION OU DE TRAITEMENT D'UNE MALADIE ASSOCIÉE À LA FORMATION INTRACELLULAIRE D'INCLUSIONS OU D'AGRÉGATS FIBRILLAIRES PROTÉIQUES

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **21.07.2006 US 832229 P**

(43) Date of publication of application:
**06.05.2009 Bulletin 2009/19**

(73) Proprietor: **Ramot at Tel-Aviv University Ltd.**
**61392 Tel Aviv (IL)**

(72) Inventors:
• **SOLOMON, Beka**
**46399 Herzliyz (IL)**
• **SHARON, Noa**
**36043 Kiryat Tivon (IL)**
• **GRINSTEIN, Oded**
**69057 Tel Aviv (IL)**

(74) Representative: **Raggers, René John**
**Exter Polak & Charlouis B.V.**
**P.O. Box 3241**
**2280 GE  Rijswijk (NL)**

(56) References cited:
WO-A-2006/050041    WO-A-2006/083795
WO-A-2006/100681    US-A1- 2004 013 647

• IVANENKOV V V ET AL: "Uptake and intracellular fate of phage display vectors in mammalian cells" BIOCHIMICA ET BIOPHYSICA ACTA. MOLECULAR CELL RESEARCH, ELSEVIER SCIENCE PUBLISHERS, AMSTERDAM, NL, vol. 1448, no. 3, 11 January 1999 (1999-01-11), pages 450-462, XP004277913 ISSN: 0167-4889
• HART ET AL: "CELL BINDING AND INTERNALIZATION BY FILAMENTOUS PHAGE DISPLAYING A CYCLIC ARG-GLY-ASP-CONTAINING PEPTIDE" JOURNAL OF BIOLOGICAL CHEMISTRY, AMERICAN SOCIETY OF BIOLOCHEMICAL BIOLOGISTS, BIRMINGHAM,, US, vol. 269, no. 17, 1994, pages 12468-12474, XP002136134 ISSN: 0021-9258
• STEFANIDAKIS M. AND KOIVUNEN E.: "Peptide-mediated delivery of therapeutic and imaging agents into mammalian cells." CURRENT PHARMACEUTICAL DESIGN, vol. 10, 2004, pages 3033-3044, XP002475346 cited in the application
• SOTO C.: "Protein misfolding and disease : protein refolding and therapy." FEBS LETTERS, vol. 498, May 2001 (2001-05), pages 204-207, XP004597906 cited in the application

Printed by Jouve, 75001 PARIS (FR)

SEQUENCE LISTING

<110> SOLOMON, Beka
SHARON, Noa

<120> METHOD FOR INHIBITING OR TREATING A DISEASE ASSOCIATED WITH INTRACELLULAR FORMATION OF PROTEIN FIBRILLAR INCLUSIONS OR AGGREGATES

<130> SOLOMON14APCT

<140> NOT YET ASSIGNED
<141> 2007-07-18

<150> 60/832,229
<151> 2006-07-21

<160> 5

<170> Patentin version 3.4

<210> 1
<211> 10
<212> PRT
<213> Artificial

<220>
<223> synthetic

<400> 1

```
Gly Gly Cys Arg Gly Asp Met Phe Gly Cys
1           5               10
```

<210> 2
<211> 12
<212> PRT
<213> Artificial

<220>
<223> synthetic

<400> 2

```
Glu Phe Gly Ala Cys Arg Gly Asp Cys Leu Gly Ala
1               5               10
```

<210> 3
<211> 4
<212> PRT
<213> Artificial

<220>
<223> synthetic

<400> 3

```
Glu Phe Arg His
1
```

<210> 4
<211> 24
<212> DNA
<213> Artificial

<220>
<223> synthetic

<400> 4
ccagcggccg ctctagaact agtg          24

<210> 5
<211> 25
<212> DNA
<213> Artificial

<220>
<223> synthetic

<400> 5
ccagtcgacc ggtcatggct gcgcc          25


**Description**

**BACKGROUND OF THE INVENTION**

Field of the Invention

The invention relates to inhibiting intracellular protein fibrillar inclusions or aggregates and dissolving preformed intracellular protein fibrillar inclusions or aggregates and to methods and pharmaceutical compositions for inhibiting or treating a disease associated with intracellular formation of protein fibrillar inclusions or aggregates.

Description of the Related Art

Plaque forming diseases are characterized by the presence of amyloid plaques deposits in the brain as well as neuronal degeneration. Amyloid deposits are formed by peptide aggregated into an insoluble mass. The nature of the peptide varies in different diseases but in most cases, the aggregate has a beta-pleated sheet structure and stains with Congo Red dye. In addition to Alzheimer's disease (AD), which includes early onset Alzheimer's disease, late onset Alzheimer's disease, and presymptomatic Alzheimer's disease, other diseases characterized by amyloid deposits are, for example, SAA amyloidosis, hereditary Icelandic syndrome, multiple myeloma, and prion diseases. The most common prion diseases in animals are scrapie of sheep and goats and bovine spongiform encephalopathy (BSE) of cattle (Wilesmith and Wells, 1991). Four prion diseases have been identified in humans: (i) kuru, (ii) Creutzfeldt-Jakob Disease (CJD), (iii) Gerstmann-Streussler-Sheinker Disease (GSS), and (iv) fatal familial insomnia (FFI) (Gajdusek, 1977; and Tritschler et al. 1992).

Prion diseases involve conversion of the normal cellular prion protein (PrPC) into the corresponding scrapie isoform (PrPSc). Spectroscopic measurements demonstrate that the conversion of PrPC into the scrapie isoform (PrPSc) involves a major conformational transition, implying that prion diseases, like other amyloidogenic diseases, are disorders of protein conformation. The transition from PrPC to PrPSc is accompanied by a decrease in $\alpha$-helical secondary structure (from 42% to 30%) and a remarkable increase in ß-sheet content (from 3% to 43%) (Caughey et al, 1991; and Pan et al, 1993). This rearrangement is associated with abnormal physiochemical properties, including insolubility in nondenaturing detergents and partial resistance to proteolysis. Previous studies have shown that a synthetic peptide homologous with residues 106-126 of human PrP (PrP106-126) exhibits some of the pathogenic and physicochemical properties of PrPSc (Selvaggini et al, 1993; Tagliavini et al, 1993; and Forloni et al, 1993). The peptide shows a remarkable conformational polymorphism, acquiring different secondary structures in various environments (De Gioia et al, 1994). It tends to adopt a ß-sheet conformation in buffered solutions, and aggregates into amyloid fibrils that are partly resistant to digestion with protease. Recently, the X-ray crystallographic studies of a complex of antibody 3F4 and its peptide epitope (PrP 104-113) provided a structural view of this flexible region that is thought to be a component of the conformational rearrangement essential to the development of prion disease (Kanyo et al, 1999). The identification of classes of sequences that participate in folding-unfolding and/or solubilization- aggregation processes may open new direction for the treatment of plaque forming disease, based on the prevention of aggregation and/or the induction of disaggregation (Silen and Agard, 1989; Frenkel et al, 1998; Horiuchi and Caughey, 1999).

Alzheimer's disease (AD) is a progressive disease resulting in senile dementia. Broadly speaking, the disease falls into two categories: late onset, which occurs in old age (typically above 65 years) and early onset, which develops well before the senile period, e.g., between 35 and 60 years. In both types of the disease, the pathology is similar, but the abnormalities tend to be more severe and widespread in cases beginning at an earlier age. The disease is characterized by two types of lesions in the brain, senile plaques and neurofibrillary tangles. Senile plaques are areas of disorganized neutrophils up to 150 mm across with extracellular amyloid deposits at the center, visible by microscopic analysis of sections of brain tissue. Neurofibrillary tangles are intracellular deposits of tau protein consisting of two filaments twisted about each other in pairs.

The principal constituent of the senile plaques is a peptide termed amyloid beta (Aß) or beta-amyloid peptide (ßAP or ßA). The amyloid beta peptide is an internal fragment of 39-43 amino acids of a precursor protein termed amyloid precursor protein (APP). Several mutations within the APP protein have been correlated with the presence of Alzheimer's disease (Goate et al, (1991), valine717 to isoleucine; Chartier Harlan et al, (1991), valine717 to glycine; Murrell et al, (1991), valine717 to phenylalanine; Mullan et al, (1992), a double mutation, changing lysine595-methionine596 to asparagine595-leucine596).

Such mutations are thought to cause Alzheimer's disease by increased or altered processing of APP to beta-amyloid, particularly processing of APP to increased amounts of the long form of beta-amyloid (i.e., Aßl-42 and Aßl-43). Mutations in other genes, such as the presenilin genes, PS1 and PS2, are thought indirectly to affect processing of APP to generate increased amounts of long form beta-amyloid (see Hardy, TINS 20, 154, 1997). These observations indicate that beta-amyloid, and particularly its long form, is a causative element in Alzheimer's disease.

Other peptides or proteins with evidence of self aggregation are also known, such as, but not limited to, amylin (Young et al, 1994); bombesin, cerulein, cholecystokinin octapeptide, eledoisin, gastrin-related pentapeptide, gastrin tetrapeptide, somatostatin (reduced), substance P; and peptide, luteinizing hormone releasing hormone, somatostatin N-Tyr (Banks and Kastin, 1992). Table 1 below provides a list of some conformational diseases related to neurodegeneration.

**Table 1**
**Conformational diseases related to neurodegeneration**

| Disorder | Protein |
| --- | --- |
| Alzheimer's disease | Amyloid-β |
| Parkinson's disease | α-Synuclein |
| Prion diseases | PrP |
| Amyotrophic lateral sclerosis | SODI |
| Spinocerebellar ataxia (SCA1) | ataxin-1 |
| Spinocerebellar ataxia (SCA3) | ataxin-3 |
| Spinocerebellar ataxia (SCA6) | calcium channel |
| Spinocerebellar ataxia (SCA7) | ataxin-7 |
| Huntington disease | Huntington |
| Entatorubral-pallidoluysian atrophy | atrophin-1 |
| Spinal and bulbar muscular atrophy | androgen receptor |
| Hereditary cerebral amyloid angiopathy | fragment of cystatin-C |
| Familial amyloidosis | Transthyretin/lysozyme |
| Frontotemporal lobe dementia | Mutant tau protein |
| British/Danish dementia | Mutant briPP protein-fragment |
| Familial encephalopathy | Mutant neuroserpin |

Publications on amyloid fibers indicate that cylindrical ß-sheets are the only structures consistent with some of the x-ray and electron microscope data, and fibers of Alzheimer Aß fragments and variants are probably made of either two or three concentric cylindrical ß-sheets (Peretz et al., 2002). The complete Aß peptide contains 42 residues, just the right number to nucleate a cylindrical shell; this finding and the many possible strong electrostatic interactions in ß-sheets made of the Aß peptide in the absence of prolines account for the propensity of the Aß peptide to form the extracellular amyloid plaques found in Alzheimer patients. If this interpretation is correct, amyloid consists of narrow tubes (nanotubes) with a central water-filled cavity. Reversibility of amyloid plaque growth *in vitro* suggests steady-state equilibrium between ßA in plaques and in solution (Maggio and Mantyh, 1996). The dependence of ßA polymerization on peptide-peptide interactions to form a ß-pleated sheet fibril, and the stimulatory influence of other proteins on the reaction, suggest that amyloid formation may be subject to modulation. Many attempts have been made to find substances able to interfere with amyloid formation. Among the most investigated compounds are antibodies, peptide composed of beta-breaker amino acids like proline, addition of charged groups to the recognition motif and the use of N-methylated amino-acid as building blocks (reviewed by Gazit, 2002).

Cyclic peptides made of alternate D and L residues form such nanotubes that kill bacteria by inserting themselves into membranes and depolarizing them (Peretz et al., 2002). There is some suggestion that some amyloid fibers might be conductors and kill cells by the same mechanism.

Aromatic compounds such as congo red that can insert themselves into gaps between helical turns might destabilize the cylindrical shells and initiate this process, but prevention would be more effective and probably easier to achieve (Peretz et al., 2002).

**Intracellular aggregates - hallmarks of conformational diseases**

Protein misfolding and conformational change are the main causes of the appearance and progress in several types of diseases (Raso et al., 2000) and a list of conformational diseases, together with their associated protein component(s) and cellular inclusion(s), is shown in Table 2 below (Goedert et al., 1998). In some cases, more than one protein is involved with a disorder, coexisting in a plaque or making its formation easier.

Table 2

| Protein fibrillar inclusions in neurodegenerative and other types of diseases | | |
|---|---|---|
| Disease | Protein component | Cellular inclusion |
| **Neurodegenerative** | | |
| Alzheimer's | τau, Aβ 42 peptide | Neurofibrillary tangles |
| Pick's | τau | Pick bodies/cytoplasmic |
| Progressive supranuclear palsy (PSP) | τau, heat shock proteins | Neurofibrillary tangles |
| Dementia with Lewy bodies | α-Synuclein | Lewy bodies/cytoplasmic |
| Parkinson's | α-Synuclein, crystallins | Neurofilaments/cytoplasmic |
| Huntington's | Expanded Glu repeats of huntingtin | Intranuclear inclusion |
| Spinocerebellar ataxias (SCA) | Expanded Glu repeats of ataxins 1,3,7 | Intranuclear inclusion |
| Transmissible spongiform encephalopathies (TSE) | Prion protein, cathepsin B | Endosome-like organelles |
| **System amyloidosis** | | |
| Diabetes type 2 | Amylin | |
| Haemodialysis related A | β-2 Microglobulin | |
| Reactive amyloidosis | Amyloid A | |
| Cystic fibrosis | CFTR protein | |

In Alzheimer's disease (AD), which represents a major problem in the Western world's aging population, the main protein component is APP, a transmembrane protein of approximately 700 amino-acid residues. In its abnormal processing, Aß (1-40) peptide is produced extracellularly and deposited as plaques. Another hallmark of AD is neurofibrillary tangles of another protein, τau (tau), which are observed in the cell. τau is a microtubule-associated protein involved in stabilizing axonal microtubules located intracellularly in neurons.

In Parkinson's disease, which is the second most common neurodegenerative disease, several proteins are implicated, α-synuclein, synphilin (an α-synuclein interacting protein) and parkin (Ciechanover, 2001). A feature of Parkinson's disease is the presence of intracellular Lewy bodies, which are found in sporadic cases of Parkinson's disease, in dementia with Lewy bodies and in the Lewy body variant of Alzheimer's disease (Chung et al., 2001). α-Synuclein is the main component of the Lewy bodies (Spillantini et al., 1997). Both α-synuclein and synphilin are required for formation of the Lewy bodies where ubiquitination of synphilin probably takes place (Ciechanover, 2001; and Chung et al., 2001.

The prion diseases, further examples of conformational diseases, a range of transmissible spongiform encephalopathies (kuru, Creuzfeldt-Jacob disease and fatal familial insomnia in humans, bovine spongiform encephalopathy in cattle, scrapie in sheep, and chronic wasting disease in deer) have many features in common with intracellular amyloidoses and most likely are 'conformational' (Soto, 2001). So far, about 20 human proteins have been found in proteinaceous deposits in various conformational diseases. These do not demonstrate any sequence or structural homology. The common event is thought to be a conformational change, leading to lack of biological function or gain of toxic activity, and possibly, formation of amyloid fibrils.

It is a matter of debate as to whether the fibrillar aggregates and amyloid plaques are the side-product of some other pathology or whether they are the main cause of the disease. Colocalization of protein aggregates with degenerating tissue and association of their presence with disease symptoms are a strong indication of the involvement of amyloid deposition in the pathogenesis of conformational diseases (Soto, 2001). In familial cases of some neurodegenerative diseases (Tables 1 and 2), evidence has been obtained for a direct link between the ability of mutated protein to form fibrils and the appearance of signs of the pathology (Goedert et al, 1998; and Conway et al., 1998). Studies with transgenic animals have also confirmed the contribution of the mutation in the amyloidogenic protein and disease pathogenesis (Soto, 2001; Scherzinger et al., 1997; and Turmaine et al., 2000).

**Similarity of amyloid formation in different proteins.**

Dobson and coauthors proposed that amyloid-fibril formation is a generic property of proteins (Guijarro et al., 1998; Fändrich et al., 2001; and Dobson, 1999). A common observation is that fibrillization starts from an intermediate state, either partially unfolded or partially folded, molten globule or native-like intermediate (Rochet et al., 2000). The parts with the α-helical structure must undergo an α- to ß-transition and the ß-strands then associate into a regular fibrillar structure.

*In vitro*, variation of solvent conditions by changing pH or adding organic solvents (Buck, 1998) can lead to partial unfolding and subsequent protein fibril formation (Chiti et al., 1999a and 1999b). *In vivo*, partial unfolding may happen as a consequence of lowered protein stability due to mutation, local change in pH at membranes, oxidative and heat stress, whereas partial folding may happen on exposure to environmental hydrophobic substances, such as pesticides (Uversky et al., 2001).

Common features of the fibrils are ß-strands (separated by 4.7 Å) running perpendicular to the long axis of the fibrils and ß-sheets extending parallel to this axis (Serpell, 2000). The ß-strands form a ß-helical twist with the usual repeat at every 115 or 250 Å (Serpell, 2000; and Ding et al., 1999).

Understanding amyloid-fibril formation may contribute to resolving some of today's most devastating diseases and, at the very least, increase the general knowledge about protein structure, folding and stability. Many properties of amyloid fibrils have emerged: a common structure for filaments and fibrils (Serpell, 2000), nucleation dependent kinetics (Lomakin et al., 1996), the role of oligomeric intermediates (Harper et al., 1999; and Walsh et al., 1999) and the existence of at least two protein conformations separated by a high energetic barrier, which behave as two macroscopic states (Ferreira et al., 2001; and Schlunegger et al., 2001).

**Familial Amyotrophic Lateral Sclerosis (fALS)**

Familial Amyotrophic Lateral Sclerosis (fALS), an inherent neurodegenerative disease in which motor neurons are impaired and lost, accounts for 10% of the ALS cases (Cudkowicz et al. 2004; and Gonzalez de Aguilar et al. 2007). In 20% of these cases, pathology is characterized by the intracellular accumulation of a mutant form of the protein Superoxide Dismutase 1 (SOD1) that results in amyloid-like filaments and aggregate formation (Bruijn et al. 1998; Watanabe et al. 2001; Taylor et al. 2002; Elam et al. 2003; Wood et al. 2003; and Matsumoto et al. 2005). Although fALS is attributed to dozens of known mutations in the SOD1 gene, which leads to the misfolded form of the SOD1 protein, it has not been proved whether the other 80% of ALS patients that lack any known mutation in the SOD1 gene suffer from SOD1 aggregates that might be a result of post-translational modifications or other environmental conditions that can force misfolding of the SOD1 protein.

Progress in ALS research was obtained after development of several transgenic mice models of the mutant human SOD1 protein. One of these models carries the G93A mutated SOD1 protein, which results in SOD1 accumulation and motor impairment (Watanabe et al. 2001; Wong et al. 2002; Julien and Kriz 2006; and Urushitani et al. 2007). These mice are the best and most common ALS research model animals.

**Therapeutic approach towards dissolving and prevention of intracellular aggregates**

Novel therapeutic approaches are being directed towards achieving one of the following goals: either to inhibit and/or reverse the conformational change, or to dissolve the smaller aggregates and disassemble the amyloid fibrils. Several successful attempts have been cited in the literature including the use of monoclonal antibodies that bind to the active conformation of the protein and thus inhibit conformational changes. In Alzheimer's disease, vaccination is on the horizon, in this case targeting the smaller oligomers and prefibrillar aggregates (Ingram et al., 2001). Soto and coworkers have designed the so called 'mini-chaperones', also termed 'ß sheet breakers' (Soto et al., 2001), which are peptides that bind to the sequence of the protein region responsible for self association. In the prion disease, similarly to Alzheimer's, trials are underway using monoclonal antibodies that prevent conformational change (Jones, 2001). Some drugs already in use for other purposes have been screened and several were found that both retard or reverse neuro-degeneration if used for early intervention, and also improve the disease state in quite desperate cases, as reported by the Prusiner's group (Korth et al., 2001). One of these drugs, quinacrine, is an anti-malarial agent and the other, chlorpromazine, is used to treat schizophrenia. Other blockers of amyloid fibril formation have been found, ranging from Congo Red derivatives, anti-cancer and antibiotic drugs to nicotine and melatonin (Findeis, 2000).

**Arginine-Glycine-Aspartate (RGD)-peptide for phage internalization into the cells**

The arginine-glycine-aspartic acid (RGD) cell adhesion sequence was discovered in fibronectin 22 years ago (Pierschbacher et al., 1984a). The surprising finding that only three amino acids would form an essential recognition site for cells in a very large protein was initially received with some skepticism. The observation was, however, soon confirmed with regard to fibronectin and then extended to other proteins. As predicted in the original paper on RGD, it turned out that the RGD sequence is the cell attachment site of many other adhesive proteins (Pierschbacher et al., 1984b; Yamada et al., 1984; Gartner et al., 1985; Plow et al., 1985; Suzuki et al., 1985; and Gardner et al., 1985). The Tat protein of the human immunodeficiency virus (HIV), in another internalization peptide, is an RGD containing protein with cell attachment activity. The interaction of Tat with cells is important because Tat can be internalized by cells, thus allowing Tat produced by one cell to enter another cell and turn on the production of latent HIV (Ensoli et al., 1990).

Proteins that contain the Arg-Gly-Asp (RGD) attachment site, together with the integrins that serve as receptors for them, constitute a major recognition system for cell adhesion. The RGD sequence is the cell attachment site of a large number of adhesive extracellular matrix, blood, and cell surface proteins, and nearly half of the over 20 known integrins recognize this sequence in their adhesion protein ligands. Some other integrins bind to related sequences in their ligands. The integrin-binding activity of adhesion proteins can be reproduced by short synthetic peptides containing the RGD sequence. Such peptides promote cell adhesion when insolubilized onto a surface, and inhibit it when presented to cells in solution. Reagents that bind selectively to only one or a few of the RGD-directed integrins can be designed by cyclizing peptides with selected sequences around the RGD and by synthesizing RGD mimics. As the integrin-mediated cell attachment influences and regulates cell migration, growth, differentiation, and apoptosis, the RGD peptides and mimics can be used to probe integrin functions in various biological systems.

**Phage binding and internalization to mammalian cells via receptor mediated endocytosis through integrin receptor via RGD cyclic peptide**

Cyclic peptides containing an RGD sequence may bind integrin molecules with a high affinity and, thus, allow internalization by a similar mechanism to invasin-mediated internalization of Y. pseudotuberculosis. An integrin-binding, RGD-containing cyclic peptide in a filamentous phage display system was fused to exogenous DNA sequences with the major coat protein gene of fd phage, gene VIII (Greenwood et al., 1991; and Hart et al., 1994). The aim of this approach was to display multiple copies of the integrin-binding peptide in order to maximize the opportunity for interactions between phage and cells. The capsid contains a hybrid mixture of wild-type pVIII and fusion pVIII subunits (Greenwood et al., 1991; and Hart et al., 1994). Hart designed DNA oligonucleotides encoding the cyclic peptide sequence GGCRGDMFGC (SEQ ID NO:1) to display on the phage in multiple copies. This peptide was originally isolated and described in a phage display library in the gene III-encoded minor coat protein of filamentous phage and demonstrated to have a high integrin-binding affinity (O'Neil et al., 1992). Bacteriophage fd particles displaying this peptide in their major coat protein subunits also have a high binding affinity for integrins.

The cyclic peptide sequence EFGACRGDCLGA (SEQ ID NO:2) presented on pVIII major coat protein of phage M13 was shown to bind mammalian cells in high efficiency (Ivanenkov et al., 1999; and Masliash et al., 2000).

## SUMMARY OF THE INVENTION

The present method provides the use of an therapeutic agent for inhibiting or treating a disease associated with intracellular formation of protein fibrillar inclusions or aggregates, the disease being a neurogenerative disease or system amyloidosis. The use involves administering to a mammalian subject in need thereof an effective amount of a therapeutic agent which is a filamentous bacteriophage which carries a peptide sequence containing a mammalian cell adhesion sequence that is displayed so as to be capable of internalizing said therapeutic agent into cells to inhibit or treat the disease.

The present invention also discloses methods for inhibiting the intracellular formation of protein fibrillar inclusions or aggregates or for disaggregating (dissolving) preformed intracellular protein fibrillar inclusions or aggregates.

The present invention further provides a pharmaceutical composition containing an effective amount of a therapeutic agent which is used as an active ingredient.

## BRIEF DESCRIPTION OF THE DRAWINGS

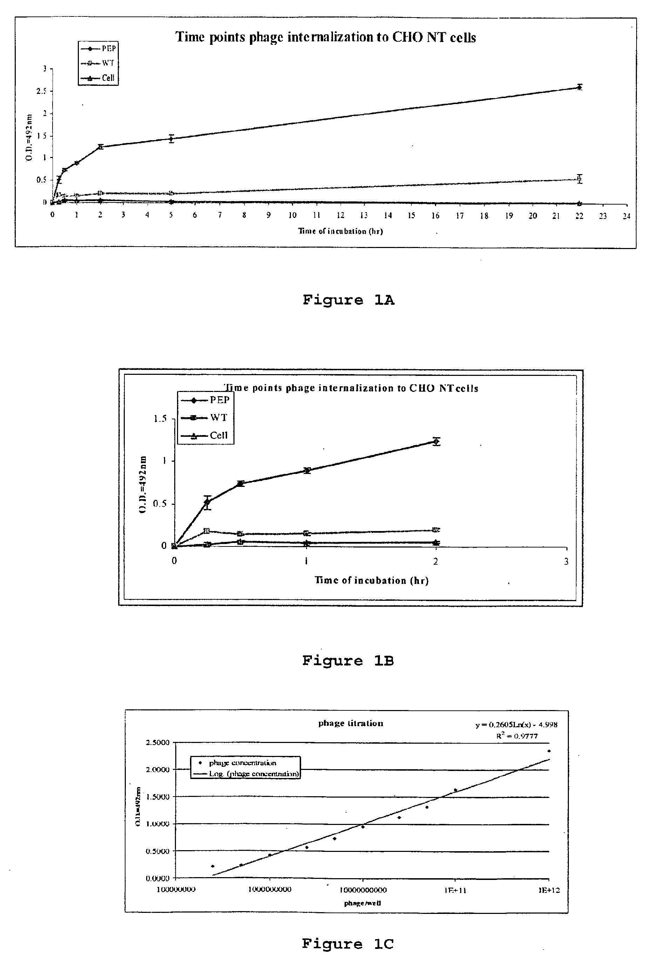

Figures 1A-1C are graphs showing quantitative analysis of internalized phage kinetics using sandwich ELISA. In Fig. 1A, CHO cells were cultured with either phage RGD (diamond), WT (squares), or PBS (triangles) at a final concentration of $6.6 \times 10^{12}$ phage per plate for several time lapses: 15 and 30 minutes, 1, 2, 5, and 22 hours. Afterwards, cells were lysed and total cell lysate was applied to a 96 microtiter plate coated with mouse anti-M13 antibody. Phage levels within the cells were detected with polyclonal rabbit anti phage antibody followed by goat anti rabbit HRP conjugated antibody. Phage concentration within the cells is proportional to optical density at 492nm with reference at 405nm as monitored by a spectrophotometer. Fig. 1B is an enlargement of Fig. 1A allowing visualization of the short time lapse up to 2 hours. Fig. 1C is a phage RGD calibration curve.

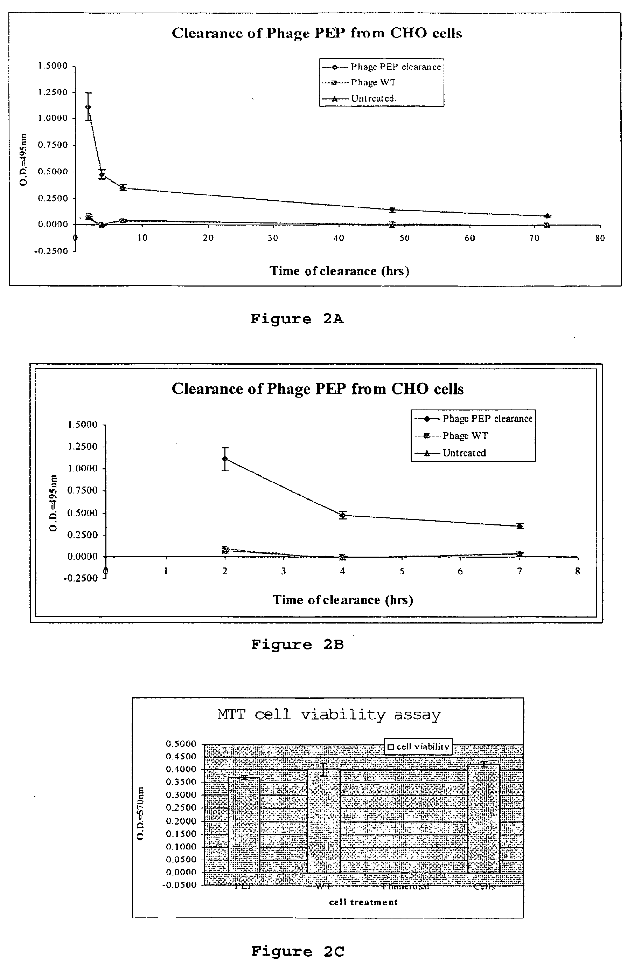

Figures 2A-2C are graphs showing the kinetics of phage clearance from CHO cells as measured by sandwich ELISA. In Fig. 2A, CHO cells were cultured with either phage RGD (diamonds), WT (squares), or PBS (triangles) at a final concentration of $6.6 \times 10^{12}$ phage per plate for 2 hours initial internalization. Afterwards, cells were washed and the medium was replaced. Cells were lysed after several time points: 2, 5, 48 and 72 hours. Cell lysates were applied to a 96 microtiter plate coated with mouse anti M13 antibody. Phage levels within the cells were detected with polyclonal rabbit anti-phage antibody followed by goat anti rabbit HRP conjugated antibody. Phage concentration within the cells is proportional to optical density measured at 492nm with reference of 405nm as monitored by a spectrophotometer. Fig. 2B is an enlargement of Fig. 2A, which allows visualization of the short time lapse up to 10 hours. Fig. 2C shows a MTT viability assay of additional plates of CHO cells that were incubated with phage RGD, WT, PBS or thimerosal, for 1 week.

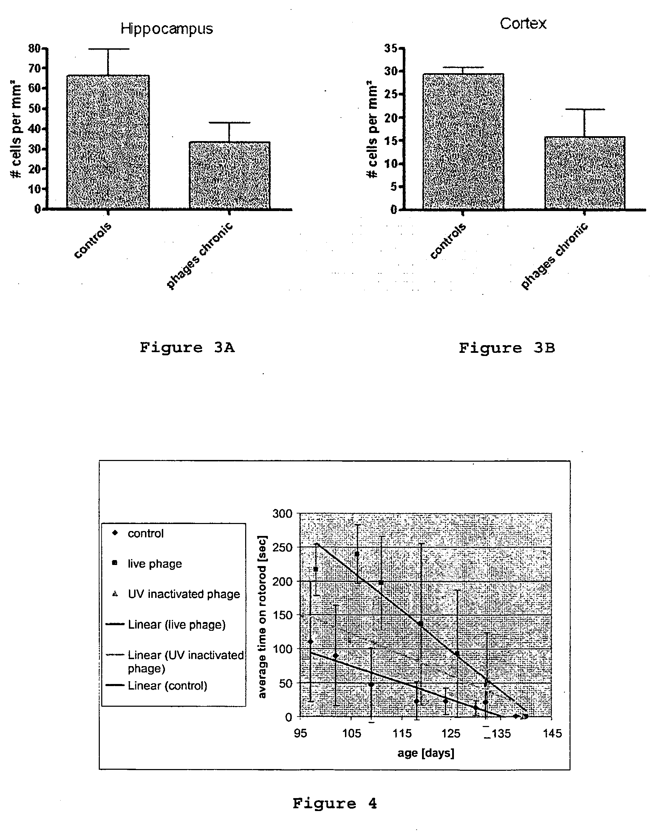

Figures 3A and 3B are graphs showing staining of Lewy bodies in the hippocampus (Fig. 3A) and cortex (Fig. 3B) of transgenic mice. Group A/control were treated with aqua bidest once per week, for 8 weeks (n=5). Group B/ phages chronic were treated with filamentous phages once per week for 8 weeks (n=5). Evaluation of α-synuclein immunoreactivity in the hippocampus and cortex (n=3/group) of -15 months of age. No effects in the acute treated group (age 8 months) treated twice a day for 3 days.

Figure 4 is a graph showing Rotorod motor performance of SOD1 mice treated with phage. The Rotorod results presented here evaluate motor performance at the estimated age of disease onset of the mice. Results are calculated as the average time of 3 runs/mouse/week. It can be seen that treated mice have better motor performance than untreated mice, and retain that ability for a longer time, which translates to delay of disease onset. Complete motor dysfunction reached end-point at about the same age in all groups.

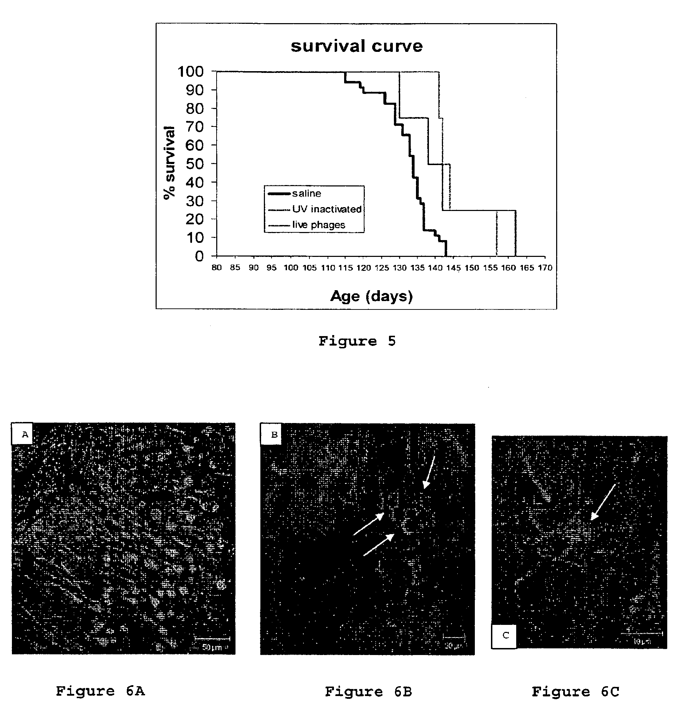

Figure 5 is a graph showing the survival rate of SOD1 ALS model mice treated with phage displaying RGD. The dark thick line shows the survival rate of untreated mice. The light thin line shows survival rate of UV-inactivated phage-treated mice, showing delay of onset of death of 26 days relative to control group and 14 day delay in death of the oldest mouse. Fifty percent survival average is estimated at 10 days longer than the control group. The dark thin line shows survival rate of live phage-treated mice, showing delay of onset of death of 15 days relative to control group and 19 day delay in death of the oldest mouse. Fifty percent survival average is estimated at 8 days longer than the control group.

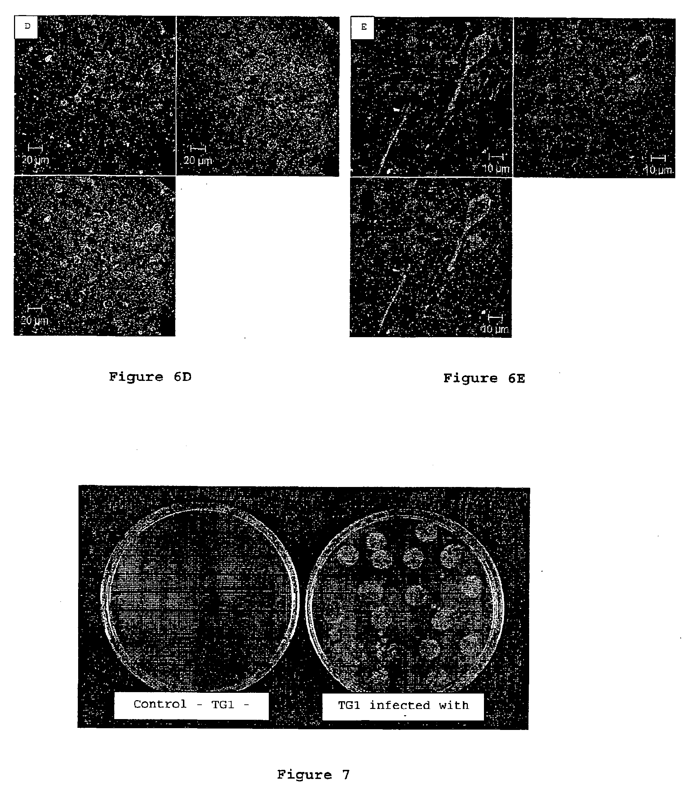

Figures 6A-6E are images from SOD1 and phage immunohistology and confocal microscope results, where Fig. 6A shows SOD1 staining of neurons in cortex using cy2 labeling of monoclonal antibody anti- human SOD1(Santa Cruz 1:500). A large number of SOD1 positive neurons can be seen in the cortex. Fig. 6B shows phage labeling using cy3 labeled rabbit polyclonal anti-M13 phage (1:500). Arrows point to some of the phage positive cortical neurons and the presence of the phages inside the neuron cytoplasm. Fig. 6C shows phage labeling of an astrocyte (by morphology only) using cy3 labeling of rabbit polyclonal anti-M13 phage (1:500). Figs. 6D and E show double-labeling of SOD1 neurons and M13 phage demonstrate phage internalization to SOD1 positive neurons and their presence in the cytoplasm.

Figure 7 are images of petri dishes showing the ability of phage to infect TG1 bacteria grown on tetracycline medium, where in the control dish, TG1 given PBS does not grow but when TG1 are given M13 phages, then they can grow on the tetracycline medium as a result of the infecting phages that confer the bacteria tetracycline resistance.

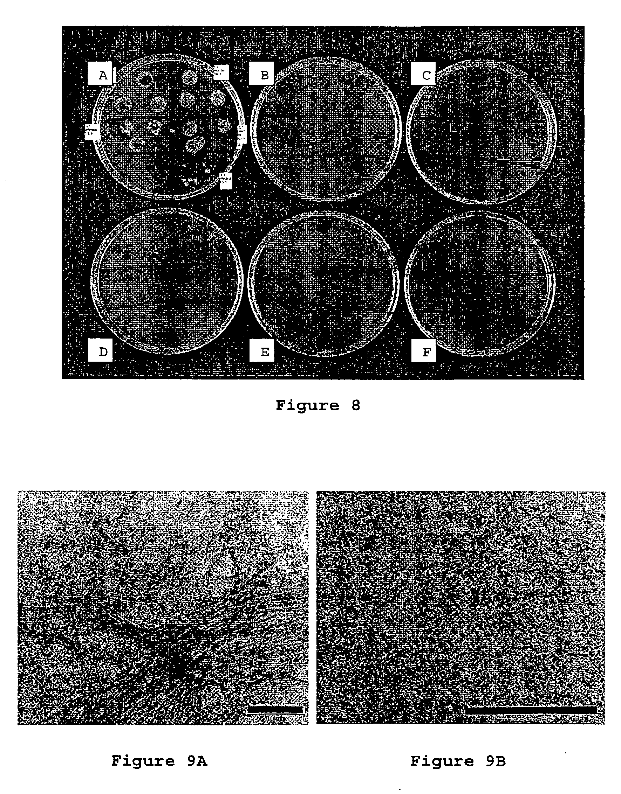

Figure 8 are images of petri dishes showing that UV irradiation of phage prevents the ability of phages to infect TG1 bacteria, where petri dish A is a positive control - TG1 bacteria infected with M13 phages, thus able to grow on tetracycline; Petri dish B - TG1 bacteria infected with M13 phages that went through one cycle of UV inactivation 200,000 μj, thus losing their ability to infect the bacteria; Petri dish C - TG1 bacteria infected with M13 phages that went through two cycles of UV inactivation 200,000 μj; Petri dish D - TG1 bacteria infected with M13 phages that went through three cycles of UV inactivation 200;000 μj; Petri dish E - TG1 bacteria infected with M13 phages that went through four cycles of UV inactivation 200,000 μj; and Petri dish F - TG1 bacteria infected with M13 phages that went through five cycles of UV inactivation 200,000 μj.

Figure 9A and 9B are electron micrographs of UV irradiated phages (bar 100 nm). Figure 9A shows wild type phages in their natural filamentous structure and Figure 9B shows UV irradiated phages. The phage filamentous structure in the UV-irradiated phages is undamaged.

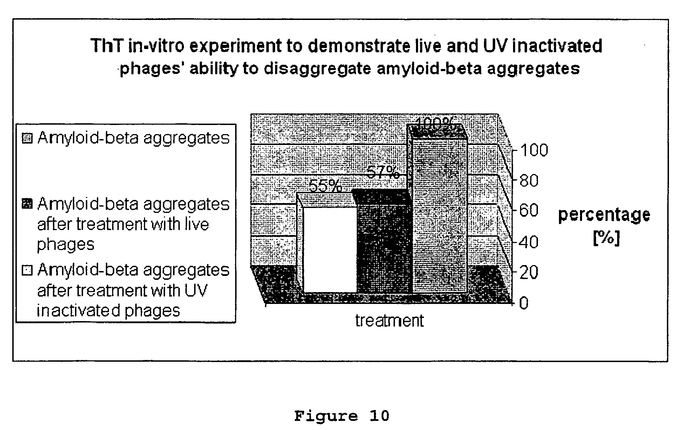

Figure 10 is a graph showing the results of a ThT assay evaluating phage interference with Aβ1-40 aggregation. The amyloid content was spectrofluorimeterically measured at 485nm wavelength following excitation at 435nm.

## DETAILED DESCRIPTION OF THE INVENTION

ß-Amyloid peptide (ßA) is one of the two hallmarks of Alzheimer's disease. This peptide forms fibrillar toxic aggregates in brain tissue that can be dissolved only by strong denaturing agents. Since these neurotoxic properties are related to peptide aggregation forms, much effort has been invested in developing a therapeutic approach towards reducing or eliminating the extent of amyloid fibrillar deposition in the brain.

Under physiological conditions, a synthetic ßA adopts an aggregated form and also shows a change from a neurite, promoting a neurotoxic effect on hippocampal neurons. Aggregation of ßA has been shown to depend on pH, peptide concentration, temperature, and time of incubation.

In the laboratory of the present inventors, filamentous phages M13, f1, and fd, which are well understood at both structural and genetic levels (Greenwood et al., 1991) were used. This laboratory first showed that filamentous bacteriophage exhibits penetration properties to the central nervous system while preserving both the inert properties of the vector and the ability to carry foreign molecules (Frenkel and Solomon, 2002).

The laboratory of the present inventors have also surprisingly discovered that filamentous phage *per se* has the ability to prevent ßA aggregation in vitro, as well as to dissolve already formed aggregates.

Filamentous bacteriophages are a group of structurally related viruses which contain a circular single-stranded DNA genome. They do not kill their host during productive infection. The phages that infect *Escherichia coli* containing the F plasmids are collectively referred to as Ff bacteriophages. They do not infect mammalian cells.

The filamentous bacteriophages are flexible rods about 1 to 2 microns long and 6 nm in diameter, with a helical shell of protein subunits surrounding a DNA core. The two main coat proteins, protein pIII and the major coat protein pVIII, differ in the number of copies of the displayed protein. While pIII is presented in 4-5 copies, pVIII is found in -3000 copies. The approximately 50-residue major coat protein pVIII subunit is largely alpha-helical and the axis of the alpha-helix makes a small angle with the axis of the virion. The protein shell can be considered in three sections: the outer surface, occupied by the N-terminal region of the subunit, rich in acidic residues that interact with the surrounding solvent and give the virion a low isoelectric point; the interior of the shell, including a 19-residue stretch of apolar side-chains, where protein subunits interact mainly with each other; and the inner surface, occupied by the C-terminal region of the subunit, rich in basic residues that interact with the DNA core. The fact that virtually all protein side-chain interactions are between different subunits in the coat protein array, rather than within subunits, makes this a useful model system for studies of interactions between alpha-helical subunits in a macromolecular assembly. The unique structure of filamentous bacteriophage enables its penetration into the brain, although it has a mass of approximately 16.3MD and may contribute to its ability to interfere with ßA fibrillization since the phage structure resemble an amyloid fibril itself.

Considering the above, the laboratory of the present inventors have examined the ability of filamentous phage to interfere with the aggregation process of ß-amyloid peptide and found that in vitro incubation of wild-type filamentous phage with ß-amyloid peptide at different time intervals, with differing ratios, leads to prevention and/or disaggregation of ß-amyloid. Moreover, the filamentous phage shows a protective effect on cell viability.

The laboratory of the present inventors have thus demonstrated the in vitro modulating effect of filamentous phage M13 on amyloid-ß peptide (AßP) aggregation, as well as the neuroprotective activity of the phage against aggregated Aß mediated toxicity. The linear structure of filamentous phages was shown to confer anti-aggregating properties against Aß, inhibiting amyloid formation and dissolving already formed aggregates both in vitro and in vivo. Modifying the phage's linear structure and rendering it spherical abolished its disaggregating as well as penetrating abilities.

The laboratory of the present inventors recently showed that due to their linear shape, filamentous bacteriophages have high permeability to different kinds of membranes. This unique structure enables their penetration to the CNS despite their high M.W. These phages were engineered to display anti-Aß antibodies and were delivered via intranasal administration into transgenic mice brains and were co-localized with Aß plaques. Recently, a similar treatment strategy against cocaine abuse was proposed using intranasal administration of an engineered filamentous bacteriophage displaying cocaine-sequestering antibodies on its surface. These phage particles were an effective vector for CNS penetration and were capable of binding cocaine, thereby blocking its behavioral effects in a rodent model (Carrera et al., 2004).

Data were obtained after incubation of the filamentous phage - ß-Amyloid fibrils with microglia cells grown on slides. If ß-amyloid does activate microglia, the phage dissolves it without activating microglia. Phage technology provides a new and practically unlimited source of the anti-aggregating agent of ß-amyloid, preventing the harmful effect of antibodies which might overactivate microglia via Fc receptors.

Bacteriophages have distinct advantages over animal viruses as gene and/or delivery vehicles. They are simple systems whose large-scale production and purification is very efficient and much cheaper than that of animal viral vectors. In addition, large segments of DNA can be efficiently packaged in phagemid vectors. Having evolved for prokaryotic infection, assembly and replication, bacteriophage can neither replicate in, nor show natural tropism for, mammalian cells. This minimizes the chances of non-specific gene delivery. Phage vectors are potentially much safer than viruses as they are less likely to generate a replication-competent entity in animal cells (Monaci et al., 2001).

The present invention provides the use of a therapeutic agent for inhibiting or treating a disease associated with intracellular formation of protein fibrillar inclusions or aggregates by administering to a mammalian subject in need thereof an effective amount of a therapeutic agent which is a filamentous bacteriophage which carries a peptide sequence comprising a mammalian cell adhesion sequence that is displayed so as to capable of internalizing the therapeutic agent into cells of the mammalian subject to inhibit or treat the disease, the disease being a neurogenerative disease or system amyloidisis.

A disease inhibited or treated by the use of the present invention refers to those diseases associated with intracellular formation of protein fibrillar inclusions or aggregates, such as intracellular neurofibrillary tangles, Pick bodies, Lewy bodies, neurofilaments, intranuclear inclusions and endosome-like organelles made up of protein components such tau, Aß1-42 peptide, heat shock proteins, α-synuclein, cystallins, expanded Glu repeats of huntingtin or ataxins, prion proteins, cathepsin B, amylin, amyloid A and ß-2 microglobulin, etc., as disclosed in Table 1. Preferably, the disease is Alzheimer's disease, Parkinson's disease, dementia with Lewy bodies, or Amyotrophic Lateral Sclerosis (ALS).

The therapeutic agent carrying the peptide sequence according to the current invention is a filamentous bacteriophage which displays the peptide sequence on its surface, preferably with the proviso that the filamentous bacteriophage does not also display an antibody or a non-filamentous bacteriophage antigen other than the peptide sequence.

The mammalian cell adhesion sequence can be any sequence which facilitates internalization as a result of cell adhesion. Preferably, the mammalian cell adhesion sequence is an Arg-Gly-Asp (RGD) cell adhesion sequence. Most preferably the RGD cell adhesion sequence comprises SEQ ID NO:1 or SEQ ID NO:2 and is cyclic. Another non-limiting example of a cell adhesion sequence is the Tat peptide from HIV.

The filamentous bacteriophage can be any filamentous bacteriophage such as M13, f1, fd, or a mixture thereof. Although M13 was used in the Examples hereinbelow, any other filamentous bacteriophage is expected to behave and function in a similar manner as they have similar structure and as their genomes have greater than 95% genome identity. The filamentous bacteriophage is preferably UV-irradiated and inactivated, where the UV-irradiation and inactivation leaves the filamentous structure of the bacteriophage undamaged so that it retains its ability to inhibit the formation of intracellular protein fibrillar inclusions or aggregates or its ability to disaggregate preformed intracellular protein fibrillar inclusions or aggregates.

When the method is used to inhibit or treat diseases which occur in the brain, the filamentous bacteriophage is preferably administered intranasally to introduce the active ingredient into the body of the recipient through an olfactory system of the recipient.

Filamentous bacteriophage M13 as the preferred embodiment of the therapeutic agent has many advantages. It is non-toxic, well established system for the expression of peptides or proteins. It stabilizes the peptides or proteins expressed on its coat proteins, is easy to scale up into large scale production, has no tropism to mammalian cells, and facilitates penetration into the brain.

The present invention also describes a method for inhibiting the intracellular formation of protein fibrillar inclusions or aggregates. This method involves causing a filamentous bacteriophage, which carries a peptide sequence containing a mammalian cell adhesion sequence that is displayed so as to be capable of internalizing the therapeutic agent into mammalian cells, to be in contact with an intracellular peptide or polypeptide capable of forming protein fibrillar inclusions or aggregates to inhibit the intracellular formation of protein fibrillar inclusions or aggregates.

The present invention further describes a method for disaggregating pre-formed intracellular protein fibrillar inclusions or aggregates. This embodiment of the present invention involves causing a filamentous bacteriophage, which carries a peptide sequence containing a mammalian cell adhesion sequence that is displayed so as to be capable of internalizing the therapeutic agent into cells, to be in contact with pre-formed intracellular fibrillar inclusions or aggregates to disaggregate the pre-formed intracellular protein fibrillar inclusions or aggregates.

The anti-aggregating or disaggregating property of the filamentous bacteriophage with respect to ßA fibril formation or disaggregation can be measured by the well-known Thioflavin T (ThT) binding assay. Disrupted formation of ßA fibril structure and disaggregation of preformed ßA fibrils are indicated by a substantial decrease in ThT fluorescence.

For purposes of this specification and the accompanying claims, the terms "subject", "patient" and "recipient" are used interchangeably. They are most preferably human but can be any mammal including mouse, rat, monkey, cow, sheep, goat, pig, horse, dog, cat, etc. which are the object of either prophylactic, experimental, or therapeutic treatment.

The terms "beta amyloid peptide" is synonymous with "ß-amyloid peptide", "ßAP", "ßA", and "Aß". All of these terms refer to a plaque forming peptide derived from amyloid precursor protein.

As used herein, "PrP protein", "PrP", "prion", refer to polypeptides which are capable under appropriate conditions, of inducing the formation of aggregates responsible for plaque forming diseases. For example, normal cellular prion protein (PrPC) is converted under such conditions into the corresponding scrapie isoform (PrPSc) which is responsible for plaque forming diseases such as, but not limited to, bovine spongiform encephalopathy (BSE), or mad cow disease, feline spongiform encephalopathy of cats, kuru, Creutzfeldt-Jakob Disease (CJD), Gerstmann-Straussler-Scheinker disease (GSS), and fatal familial insomnia (FFI).

As used herein, the term "disaggregating" refers to solubilization (dissolving) of aggregated proteins typically held together by non-covalent bonds.

The term "treating" is intended to mean substantially inhibiting, slowing or reversing the progression of a disease, substantially ameliorating clinical symptoms of a disease or substantially preventing the appearance of clinical symptoms of a disease.

Also as used herein, the term "plaque forming disease" refers to diseases characterized by formation of plaques by an aggregating protein (plaque forming peptide), such as, but not limited to, beta-amyloid, serum amyloid A, cystatin C, IgG kappa light chain or prion protein, in diseases such as, but not limited to, early onset Alzheimer's disease, late onset Alzheimer's disease, presymptomatic Alzheimer's disease, SAA amyloidosis, hereditary Icelandic syndrome, senility, multiple myeloma, and to prion diseases that are known to affect humans, such as for example, kuru, Creutzfeldt-Jakob disease (CJD), Gerstmann-Straussler-Scheinker disease (GSS), and fatal familial insomnia (FFI) and animals, such as, for example, scrapie and bovine spongiform encephalitis (BSE).

Because most of the amyloid plaques (also known as amyloid deposits) associated with the diseases described hereinabove are located within the brain, any proposed treatment modality for extracellular plaques must demonstrate an ability to cross the blood brain barrier (BBB) as well as an ability to dissolve amyloid plaques. Normally, the average size of molecules capable of penetrating the BBB is approximately 2 kDa.

An increasing body of evidence shows that olfactory deficits and degenerative changes in the central olfactory pathways are affected early in the clinical course of AD. Moreover, the anatomic patterns involved in AD suggest that the olfactory pathway may be the initial stage in the development of AD.

Olfactory receptor neurons are bipolar cells that reside in the epithelial lining of the nasal cavity. Their axons traverse the cribriform plate and project to the first synapse of the olfactory pathway in the olfactory bulb of the brain. This configuration makes them a highway by which viruses or other transported substances may gain access to the CNS across the BBB.

As previously shown, intranasal administration (Mathison et al, 1998; Chou et al, 1997; Draghia et al, 1995) enables the direct entry of viruses and macromolecules into the cerebrospinal fluid (CSF) or CNS.

Use of olfactory receptor neurons as a point of delivery for an adenovirus vector to the brain is reported in the literature. This method reportedly causes expression of a reporter gene in the brain for 12 days without apparent toxicity (Draghia et al, 1995).

A pharmaceutical preparation according to the present invention includes, as an active ingredient, an effective amount of a therapeutical agent which carries a peptide sequence that is displayed so as to be capable of internalizing the therapeutic agent into cells.

The preparation according to the present invention can be administered to an organism *per se*, or in a pharmaceutical composition where it is mixed with suitable carriers or excipients.

As used herein a "pharmaceutical composition" refers to a preparation of one or more of the active ingredients described herein with other chemical components such as physiologically suitable carriers and excipients. The purpose of a pharmaceutical composition is to facilitate administration of a compound to an organism.

Herein the term "active ingredient" refers to the preparation accountable for the biological effect.

Hereinafter, the phrases "physiologically acceptable carrier" and "pharmaceutically acceptable carrier" which may be interchangeably used refer to a carrier or a diluent that does not cause significant irritation to an organism and does not abrogate the biological activity and properties of the administered compound. An adjuvant is included under these phrases.

Herein the term "excipient" refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils and polyethylene glycols.

Techniques for formulation and administration of drugs may be found in "Remington's Pharmaceutical Sciences," Mack Publishing Co., Easton, PA, latest edition .

Suitable routes of administration may, for example, include oral, rectal, transmucosal, especially transnasal, intestinal or parenteral delivery, including intramuscular, subcutaneous and intramedullary injections as well as intrathecal, direct intraventricular, intravenous, intraperitoneal, intranasal, or intraocular injections.

Alternatively, one may administer a preparation in a local rather than systemic manner, for example, via injection of the preparation directly into the brain of a patient.

Pharmaceutical compositions of the present invention may be manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with the present invention thus may be formulated in conventional manner using one or more physiologically acceptable carriers comprising excipients and auxiliaries, which facilitate processing of the active ingredients into preparations which, can be used pharmaceutically. Proper formulation is dependent upon the route of administration chosen.

For injection, the active ingredients of the invention may be formulated in aqueous solutions, preferably in physiologically compatible buffers such as Hank's solution, Ringer's solution, or physiological salt buffer. For transmucosal administration, penetrants appropriate to the barrier to be permeated are used in the formulation. Such penetrants are generally known in the art.

For oral administration, the therapeutic agent can be formulated readily by combining the active ingredient with pharmaceutically acceptable carriers well known in the art. Such carriers enable the therapeutic agent to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carbomethylcellulose; and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active ingredient doses.

Pharmaceutical compositions, which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the active ingredients may be dissolved or suspended in suitable liquids, such as fatty oils, liquid paraffin, or liquid polyethylene glycols. In addition, stabilizers may be added. All formulations for oral administration should be in dosages suitable for the chosen route of administration.

For buccal administration, the compositions may take the form of tablets or lozenges formulated in conventional manner.

For administration by nasal inhalation, the active ingredients for use according to the present invention are conveniently delivered in the form of an aerosol spray presentation from a pressurized pack or a nebulizer with the use of a suitable propellant, e.g., dichlorodifluoromethane, trichlorofluoromethane, dichloro-tetrafluoroethane or carbon dioxide. In the case of a pressurized aerosol, the dosage unit may be determined by providing a valve to deliver a metered amount. Capsules and cartridges of, e.g., gelatin for use in a dispenser may be formulated containing a powder mix of the compound and a suitable powder base such as lactose or starch.

The preparations described herein may be formulated for parenteral administration, e.g., by bolus injection or continuous infusion. Formulations for injection may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

Pharmaceutical compositions for parenteral administration include aqueous solutions of the active preparation in water-soluble form. Additionally, suspensions of the active ingredients may be prepared as appropriate oily or water based injection suspensions. Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents which increase the solubility of the active ingredients to allow for the preparation of highly concentrated solutions.

Alternatively, the active ingredient may be in powder form for constitution with a suitable vehicle, e.g., sterile, pyrogen-free water based solution, before use.

The preparation of the present invention may also be formulated in rectal compositions such as suppositories or retention enemas, using, e.g., conventional suppository bases such as cocoa butter or other glycerides.

Pharmaceutical compositions suitable for use in the context of the present invention include compositions wherein the active ingredients are contained in an amount effective to achieve the intended purpose. More specifically, a therapeutically effective amount means an amount of active ingredients effective to prevent, alleviate or ameliorate symptoms of disease or prolong the survival of the subject being treated. It can also mean an effective amount that inhibits or reduces the intracellular formation of protein fibrillar inclusions or aggregates or disaggregates/dissolves preformed intracellular protein fibrillar inclusions or aggregates.

Determination of a therapeutically effective amount is well within the capability of those skilled in the art, especially in light of the detailed disclosure provided herein.

For any preparation used in the methods of the invention, the therapeutically effective amount or dose can be estimated initially from *in vitro* and cell culture assays. For example, a dose can be formulated in animal models to achieve a desired effect. Such information can be used to more accurately determine useful doses in humans.

Toxicity and therapeutic efficacy of the active ingredients described herein can be determined by standard pharmaceutical procedures *in vitro*, in cell cultures or experimental animals. The data obtained from these in vitro and cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition. (See, e.g., Fingl et al, in "The Pharmacological Basis of Therapeutics", Ch. 1 p.1 (1975)).

Dosage amount and interval may be adjusted individually to provide plasma or brain levels of the filamentous bacteriophage which are sufficient to prevent aggregation or to disaggregate existing aggregates (minimal effective concentration, MEC). The MEC will vary for each preparation, but can be estimated from in vitro data. Dosages necessary to achieve the MEC will depend on individual characteristics and route of administration. Binding assays can be used to determine plasma concentrations.

Dosage intervals can also be determined using the MEC value. Preparations should be administered using a regimen, which maintains plasma levels above the MEC for 10-90% of the time, preferable between 30-90% and most preferably 50-90%.

Depending on the severity and responsiveness of the condition to be treated, dosing can be of a single or a plurality of administrations, with course of treatment lasting from several days to several weeks or until cure is effected or diminution of the disease state is achieved.

The amount of a composition to be administered will, of course, be dependent on the subject being treated, the severity of the affliction, the manner of administration, the judgment of the prescribing physician, etc.

Compositions of the present invention may, if desired, be presented in a pack or dispenser device, such as an FDA approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accommodated by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert. Compositions comprising a preparation of the invention formulated in a compatible pharmaceutical carrier may also be prepared, placed in an appropriate container, and labeled for treatment of an indicated condition, as if further detailed above.

Having now generally described the invention, the same will be more readily understood through reference to the following examples which are provided by way of illustration and are not intended to be limiting of the present invention.

## EXAMPLE 1

### Alzheimer's Disease Experiments

### MATERIALS AND METHODS

### Electron microscopy (EM)

*Filamentous phage interaction with amyloid ß-peptide (AßP).*

Phages and Aß samples were adhered to formvar coated carbon evaporated on 200# grids for 10 min and blotted. To discriminate between the filamentous phage particles and the Aß fibrils, immunolabeling with monoclonal antibody (mAb) 196 raised against EFRH amino acid residues (SEQ ID NO:3) of AßP (produced in the laboratory of the present inventors) was performed to detect Aß and rabbit polyclonal anti M13 (produced in our lab) to label the phages. The immunolabeling was visualized by 12 nm gold conjugated goat anti-mouse antibody and 6 nm gold anti-rabbit antibodies, respectively (Electron Microscopy Sciences, Washington). Samples were incubated with 1st antibody for 10 min, washed 5 times with 0.1% BSA/TBS, then incubated with 2nd antibody for 30 min, washed, negatively stained with aqueous uranyl acetate (2%wt/vol) (Sigma) and observed under JEOL 1200 EX electron microscope (Ingram, 2001).

The ability of the wild type M13 phage to prevent AßP aggregation was demonstrated by incubating AßP1-40 (97 $\mu$M) with different amounts of phages ($2\times10^{12}$phages=110 nM, $2\times10^{10}$phages=1.1 nM, and $2\times10^{8}$phages = 0.01 nM) for 9 days at 37°. Addition of PBS served as control. Samples of 10 $\mu$l from AßP solution with or without phages were processed as described above.

For disaggregation experiments, AßP1-40 dissolved in PBS was incubated at 37°C for 9 days to form aggregates. Phages ($1\times10^{12}$ phages=55 nM, $1\times10^{10}$ phages= 0.55 nM) were added to the samples (Aβ1-40 97 $\mu$M), co-incubated for 16 h at 37°C and processed for EM analysis.

### Construction of phage RGD

*Preparation of competent E.coli.*

Different *E. coli* strains (K91Kan, DH5$\alpha$ and XL-1 blue) were grown in 2ml of 2YT media (containing 100 $\mu$g/ml Kanamycin, 20 $\mu$g/ml Tetracycline or no antibiotic respectively) at 37°C while shaking (250 RPM) overnight. Then, 600 $\mu$l of overnight culture was transferred to 60ml SOB media and grown to an early logarithmic state (O.D. (600nm) - 0.3). The bacteria grown were incubated on ice for 10 minutes and then centrifuged at 5000 rpm for 10 minutes at 4°C. The bacterial pellet was resuspended in 20ml CCMB and incubated for 20 minutes on ice followed by 10 minutes centrifugation at 5000rpm at 4°C. The precipitated bacteria were resuspended in 5ml CCMB, aliquoted and stored at - 70°C, until use.

**Cloning of phage RGD**

*F88-4 vector purification and cleavage.*

The f88-4 vector was isolated from a "type 88" fd library clone (provided by George Smith) using DNA midi purification kit. F88-4 vector was digested by HindIII and PstI restriction enzymes. Total reaction volume of 40 μl containing 15μg of dsDNA, 1μl PstI and 1μl HindIII, 4μl number 3 restriction enzyme buffer X10, was incubated for 2 hours at 37°C, followed by inactivation at 70°C for 5 minutes. Afterwards, 1μl of Antarctic Phosphatase was added to the digested vector in order to remove the 5'-phosphate groups from DNA for 30 minutes at 37°C. This product was electrophoresed in 0.7% agarose gel. The linear processed vector was purified from gel using DNA extraction kit.

*Insert preparation: phosphorylation of insert primers and annealing.*

Two complementary primers, 50 pMol each, were mixed with 2.5μl T4 kinase buffer X10, 1μl T4 kinase and 16.5μl DDW and incubated for 2 hours at 37°C, followed by kinase inactivation for 10 minutes at 70°C. Then, 20 pmol of each primer was mixed to a total volume of 100μl and incubated for 5 minutes at 95°C. A gradual lowering of the temperature was then applied to allow primers annealing. The annealed primers (insert) at this stage are dsDNA consisting of HindIII and PstI sticky ends.

*Ligation and transformation.*

The processed vector and annealed primers (insert) were ligated at a molar ratio 1:3 respectively, as follows: 100ng of vector, 1.423ng insert, 1.5μl T4 DNA ligase buffer X10 and 1μl T4 DNA ligase were added to total volume of 15μl and incubated for 2 hours at room temperature followed by overnight incubation at 4°C. In order to evaluate the vector self-ligation rates, additional mixture was prepared consisting of the vector only. The ligation products were transformed into competent K91Kan bacteria using heat shock procedure according to the following: 10μl of ligation mix was added to 100μl of thawed bacteria and incubated on ice for 30 minutes followed by 2 minutes incubation at 42°C and an immediate additional incubation on ice for 5 minutes. Then, 1ml of 2YT was added to the bacteria and the culture was grown at 37°C while shaking at 150rpm for 1 hour to express antibiotic resistance genes. Bacteria were seeded in two dilutions on 2YT plates containing 100μg/ml Kanamycin and 20μg/ml Tetracycline and grown at 37°C overnight.

*Identification of positive clones.*

Transformed colonies that had grown on Kan/Tet plates were subjected to colony PCR. Each colony was mixed with 7μl of ready mix, 1μl (10pmol) of each primer (the insert primer served as the forward primer and primer complementary to pVIII of f88-4 vector served as the reverse primer) and 5μl sterile ddH2O followed by a PCR reaction as follows: 95°C for 6 minutes then 29 cycles of: 30 seconds at 94°C, 30 seconds at 55°C and 1 minute at 72°C. Final strands extention was performed by additional 5 minutes incubation at 72°C. The PCR products were then applied to a 2% agarose gel in order to detect 35bp bands indicating that the RGD insert was indeed ligated to the vector. Positive colonies were then sequenced using reverse primer that was used in PCR.

*Phage production.*

K91Kan E.coli transfected with f88-4 vector containing PEP insert as well as the wild-type clone (which is the "naked" f88-4 vector), were grown in 2ml of 2YT media, including 100μg/ml Kanamycin and 20μg/ml Tetracycline overnight at 37°C, 250rpm. One ml of the overnight growing starter was added to 1 liter of 2YT media, including 100μg/ml Kanamycin, 20μg/ml Tetracycline and 1mM IPTG and was incubated overnight at 37°C, 250rpm. The next day, the inoculum was centrifuged at 6500rpm at 4°C for 20 minutes in order to eliminate bacteria. Supernatant was collected and incubated with PEG/NaCl in 5:1 (v/v) ratio overnight at 4°C to enable phage precipitation. Phages were then precipitated by one hour centrifugation at 9000 rpm at 4°C. After the supernatant was discarded, the precipitate was resuspended in 20ml of sterile PBS and precipitated once again by overnight incubation with PEG/NaCl 1:5 ratios at 4°C. Phage particles were precipitated by one hour centrifugation at 9000 rpm at 4°C, resuspended in 1ml sterile PBS and then filtrated using 0.45μ filter tip to eliminate anytraces of bacteria. The recovered phage particles concentration was determined according to the absorbance at 269nm and 320nm as reference, measured by spectrophotometer, according to the following formula:

$$\text{Phage/ml} = \frac{[\text{O.D.}_{(269nm)} - \text{O.D.}_{(320nm)}] \cdot 6 \cdot 10^{16}}{\text{Vector size (bp)}}$$

*Detection of phage production using enzyme linked immunosorbent assay (ELISA).*

ELISA 96 microtiter plates were coated with several concentrations of phage RGD or phage WT, diluted in coating buffer in a total volume of 50$\mu$l per well. After 2 hours incubation at 37°C, the plate was washed twice with PBST and twice with PBS. The plate was then blocked with 3% milk in PBS (200$\mu$l per well) for 3 hours at 37°C followed by washes, as described above. Rabbit anti-M13 50$\mu$l polyclonal antibody diluted 1:1000 in 1% milk was washed and incubated at 37°C for 1.5 hours followed by washes, as described. Goat anti-rabbit HRP conjugated antibody (50$\mu$l) was added and incubated at 37°C for 1 hour followed by washes as described. The plate was developed using 50$\mu$l of the substrate O-phenylenediamine (OPD) (15mg OPD in 7.5ml citrate buffer and adding 3$\mu$l of 30% H2O2) to each well. Reaction was stopped using 25$\mu$l of 4N HCL. Color density was monitored by absorption in 492nm with reference of 405 nm using an ELISA reader.

*Quantitative analysis of internalized phage kinetics using Sandwich ELISA.*

In order to determine the exact time of phage internalization and to quantify the amount of internalized phage at several short time-points, the phage were incubated with CHO cells similar to that described, except that in this assay the time-points were for 15 and 30 minutes, 1, 2, 5 and 22 hours. The cells were lysed and assayed for protein concentration. Ninety six well microtiter plates were coated with mouse anti-M13 monoclonal antibody diluted 1:250 in coating buffer, 50$\mu$l per well and incubated overnight at 4°C. The next day, the plate was washed twice with PBST, followed by two washes with PBS and after normalization for equal protein concentration in total volume of 50$\mu$l 1% milk in PBS, were added in triplicate. Phage calibration curve was done using the same production of phage RGD that was administered to cells in this procedure with several sequential dilutions in a total volume of 50$\mu$l 1% milk in PBS. Samples were incubated for 2h at 37°C, followed by two washes with PBST then two washes with PBS. The plate was incubated with 3% milk in PBS 100$\mu$l per well at 4°C overnight. The next day, after one wash with PBS, rabbit anti-M13 polyclonal antibodies, diluted 1:1500 in 1% milk, were added, 50$\mu$l per well for 1.5 hours at 37°C. After the plate was washed twice with PBST and twice with PBS, goat anti rabbit HRP conjugated secondary antibody 1:2500 diluted in 1% milk was added 50$\mu$l per well and incubated for 1h at 37°C. Unbound secondary antibody was removed by washing the wells twice with PBST and twice with PBS. The plate was developed using 50$\mu$l of the substrate O-phenylenediamine (OPD) (15mg OPD in 7.5ml citrate buffer and adding 3$\mu$l of 30% $H_2O_2$) to each well. Reaction was stopped using 25$\mu$l of 4N HCl. Color density was monitored by absorption at 492nm with reference at 405 nm using an ELISA reader.

*Quantification of phage clearance kinetics in CHO cells using sandwich ELISA.*

To determine phage clearance kinetics after initial internalization to CHO cells, clearance assay was performed. This assay was based on the fact that most of the phage PEP particles internalize to the cells after about 2 hours at most. $5 \times 10^5$ cells were seeded in 10 cm plates in CHO growth medium (containing 10% sera) at 37°C, 90% relative humidity and 5% $CO_2$. At 90% confluence, cells were washed with sterile PBS and growing sera free media was added to the cells. After at 2 hours incubation, cells were administered with $6 \times 10^{12}$ phage RGD or WT or with PBS as control (untreated). The phage particles were incubated with the cells for 2 hours after which cells were thoroughly washed 3 times with PBS. Sera free media was added to cells and the cells were lysed after several time intervals: 2h, 5h, 48h and 72h. Cell extracts were assayed for protein concentration. Quantitative ELISA for phage clearance from cells was similarly performed. In order to confirm that phage clearance was not a result of cell death, MTT assay was performed on additional 10cm plates of CHO cells that were incubated with phage RGD or WT or with PBS for 1 week.

*Immunofluorescence of internalized phage RGD localization within the cell.*

For specific localization within the cells of the internalized phage, colocalization of cell organelles with phage particle was performed using specific antibodies and plasmid consisting of intracellular markers. CHO cells were detached from the plate bottom, counted by Trypan Blue method and cultured on a sterile 24-well plate with 13mm $\varnothing$ glass coverslips, $5 \times 10^4$ cells per well in CHO growth medium (containing 10% sera) (700$\mu$l per well) at 37°C, 90% relative humidity and 5% $CO_2$. At approximately 60% confluent, cells were transfected with 1$\mu$g DNA of either EEA1-GFP (early endosomes marker), Lgp-120 - YFP (lysosomes marker), GalT - GFP (Golgi marker) or Sec61-GFP (E.R. marker), under the CMV promoter. Transfection was carried out with FuGENE 6 transfection reagent or with TransIT-LT1 transfection reagent according to manufacture's instructions and the plate was held in silver paper. After 24 hours, the cells were washed thoroughly and incubated with $10^{11}$ phage RGD, WT or PBS per well, for 24 hrs in sera free media. The next day, the cells were washed 3 times with sterile PBS and fixed with 500μl of 4% paraformaldehyde per well for 30 min at 40°C followed by 3 washes with 700μl of 1% NH4Cl for 5 minutes each. Cells were permeabilized using 200μl 1% saponin (unpermeabilized cells were washed with PBS) at RT for 15 minutes followed by 3 thorough washes with PBS. Blocking was done using blocking buffer (unpermeabilized wells were washed with blocking buffer without saponin) for 15 min in RT. First antibody mouse anti-M13 200μl diluted 1:500 in blocking buffer was added to each well and incubated at RT for 1h. Then cells were washed 3 times with 1% saponin (unpermeabilized wells were washed with PBS) and secondary antibody goat anti-mouse cy3 conjugated was added 1:500 in blocking buffer for 1h at RT. Cells were then washed 4 times with PBS. A ProLong anti-fade solution (mounting media) was added to a carrying glass and the coverslip (containing the cells) was applied over it. After 2 days at 40°C, cells were visualized using confocal microscope.

*Immunofluorescence of intracellular pathway of internalized phage RGD.*

To reveal phage pathway within the cells in correlation with specific endocytic organelles and at several time-points, cells were transfected with plasmid containing EEA1-GFP (early endosomes marker) or Lgp-120-YFP (lysosomes marker) under the CMV promoter. Phage particles were applied to transfected cells as described; however, in this procedure the time of incubation was: 15min, 30min, 1hour, 2hours and 5 hours. After the incubations, the cells were washed thoroughly with sterile PBS and the cells were fixated, stained and visualized as described.

## RESULTS

### Anti-aggregating properties of filamentous phage

*In vitro experiment.*

ThT experiments indicate that the disaggregating activity of the phage is more efficient than its ability to prevent extracellular fibril formation. A decline of 26% in AßP aggregation was observed when the peptide Aß was incubated in the presence of filamentous phage, while the addition of phages to preaggregated Aß resulted in a 45% reduction in amyloid fibrils.

*In vivo experiment.*

Disaggregating properties of the phages were shown *in vivo* by intracerebral injection of phages into transgenic mice over-expressing human amyloid precursor protein. The ability of filamentous phage to bind Aß plaques of transgenic mice was demonstrated by immunohistochemical staining of AD model brain sections The filamentous phage family (Ff), composed of M13, f1, and fd, is well studied, both structurally and genetically. Having evolved for prokaryotic infection, assembly and replication, the bacteriophage lacks tropism for mammalian cells. Their filamentous structure (900 nm long, 7 nm diameter) enables their penetration into the CNS. Ff virion consists of a singe-stranded DNA genome packaged in a tube comprised of the major coat protein pVIII and closed at the ends by four or five copies of each of four species of minor coat proteins. The laboratory of the present inventors has found that the linear structure of filamentous phages confers anti-aggregating properties against AßP. Modifying the phage's linear structure and rendering it spherical abolished its disaggregating abilities. Filamentous phage interferes with AßP aggregation process and dissolves existing Aß fibrils, suggesting therapeutic relevance of these findings to AD treatment, as well as to other amyloidogenic diseases.

*Phage-RGD internalization.*

Phage - RGD were internalized into CHO cells as early as 15 minutes after administration (Fig. 1B, diamonds). High levels of phage RGD were detected within 2 hours incubation with cells and continued to internalize until the time lapse of 22 hours, the last time point examined (Fig. 1A, diamonds). In contrast, phage WT showed only a moderate internalization after long incubation of 22 hours (Fig. 1A, squares) and until that time point gives the same measurements as untreated cells at all time points measured. Even so, its internalization at this time is equivalent to RGD internalization within only 15 minutes. After evaluation of the calibration curve (Fig. 1C), O.D.492 = 0.5 is similar to $2x10^9$ phage particles. It may be assumed that the amount of internalized phage RGD after 15 minutes incubation with cells is $2x10^9$. Error bars represent standard deviation values calculated from three independent experiments, with three repeats each.

A significant colocalization of phage RGD with cells that were transfected with EEA1- GFP, an early endosome marker fused to GFP or with Lgp120-YFP a lysosome marker, was observed. However, when other cell organelles were examined, the ER protein sec61-GFP and the golgi protein GalT - GFP, no colocalization of phage was detected. These results show that the phage RGD indeed internalizes through the endocytic pathway mechanism of internalization.

**Experiments in Transgenic (Tg) mouse model of AD**

Tg mice, wild-type human α-synuclein was expressed under the regulatory control of the platelet-derived growth factor-ß (PDGF-ß) promoter. This promoter was chosen because it has been successfully used to target the expression of other human proteins to neurons in transgenic models of neurodegenerative disease (Masliah et al., 2000).

A 1480-base pair (bp) fragment of 5'-flanking region of the human PDGF-ß chain gene was isolated from the psisCAT plasmid (a gift from T. Collins, Harvard Medical School) and placed upstream of a Not I-Sal I fragment consising from 5' to 3' of an SV40 splice, 423 bp of human cDNA encoding full-length wild-type α-synuclein, and SV40 sequence from the pCEP4 vector (Invitrogen) providing a polyadenylate signal. The resulting fusion gene was freed of vector sequences, purified, and microinjected into one-cell embryos (C57BL/6 x DBA/2 F2) according to standard procedures. Thirteen transgenic founders were identified by slot-blot analysis of tail DNA and bred with wild-type C57BL/6 x DBA/2 F1 mice to establish transgenic lines. Transgenic offspring were identified by polymerase chain reaction (PCR) analysis of tail DNA. Genomic DNA was extracted and amplified in 30 cycles (93°C for 30 s, 57°C for 30 s, 72°C for 1.5 min) with a final extension at 72°C for 5 min. Primers were as follows:

5'-CCAGCGGCCGCTCTAGAACTAGTG(sense; SEQ ID NO:4) and

5'-CCAGTCGACCGGTCATGGCTGCGCC (antisense; SEQ ID NO:5)

**Staining of Lewy bodies**

The antibody to human α-synuclein also recognized the characteristic intracytoplasmic inclusions found in Lewy body disease Human α-synuclein immunoreactive inclusions were most abundant in transgenic mice from the highest expresser line and were not detected in nontransgenic controls. In the transgenic mice, the inclusions were most frequently seen in neurons in the deeper layers of the neocortex, the CA3 region of the hippocampus, and the olfactory bulb and occasionally in the substantia nigra. These regions are also typically affected in patients with Lewy body disease (Masliah et al., 2000).

**DISCUSSION**

Filamentous phage M13 has many advantages for use. It is a non-toxic, well established system for the expression of peptides or proteins, stabilizes the proteins or peptides expressed on its coat proteins, consists of an easy large scale production and has no tropism to mammalian cells. It also has been shown in our lab that filamentous phage enables brain penetration.

A delivery system that internalizes into mammalian cells based on the filamentous phage M13 was used in the experiments in this example. The EFGACRGDCLGA sequence (SEQ ID NO:2), consisting of a consensus core containing Arginine-Glycine-Aspartate (an R-G-D core) that is the minimal amino acid sequence required for binding the αv integrin family (Ivanenkov et al., 1999a and 1999b). The RGD core was displayed on the phage coat proteins encircled by two cysteines, thus facilitating a cyclic presentation of the RGD core peptide. Generation of a cyclic peptide results in higher affinity compared with the noncyclic peptide and allows target specificity. Moreover, the cyclic peptide presentation grants a higher stability *in vivo* (Stefanidakis and Koivunen, 2004).

Thus, allowing a multivalent peptide presentation of -150 copies on phage surface, such presentation gave rise to an active cellular import receptor mediated endocytosis (RME) system mediated by binding of α-integrin receptors.

Using immunofluorescent labeling, phage RGD binding and internalization to mammalian CHO cells in comparison to WT phage was observed. CHO cells were selected as a cellular model to characterize phage with mammalian cells. These cells are a common and well-established cellular model and have the advantage of being adhesive. Integrins appear in large amounts on this cell surface as opposed to non-adhesive models, allowing them to interact with intracellular α-actin for binding and internalization. Dose-dependent internalization of phage RGD administered to cells was demonstrated, so that when higher amounts of phage were administered to cells, higher internalization was observed.

Some intracellular targeting systems are cytotoxic to cells especially when administered in high doses (Mountain, 2000; Lee and Jameson, 2005). Therefore, the influence of phage administration on CHO cells by two different colorimetric experiments was examined. Cell death was inspected after phage RGD or WT was administered to cells by Trypan blue count and cell viability was evaluated by MTT assay. This assay was performed in order to assure that the phage presence inside the cells did not have any adverse effects after 2 days incubation. In both these experiments, cells administered with phage RGD or phage WT showed similar viability to untreated cells, and it can be seen that these two reciprocal assays gave similar results.

To reveal the fate of phage RGD within the cell, find its specific localization and underline its pathway within the cell, both intracellular phage and cell organelles were double-labeled. In different times of incubation, the phage RGD intracellular pathway according to the endocytic pathway could be revealed: the phage was located in the early endosomes at the first 15 minutes. After 2 hours of incubation, more phage particles were located within the endosomes and within 2 to 5 hours, most of the phage particles were located at the lysosomes, which points to the endocytic pathway of internalization.

To conclude, the present inventors have established here a system for intracellular targeting into mammalian cells. An internalized phage consisting of a RGD core peptide that enables intracellular internalization with high avidity and affinity was constructed. The system was characterized for both internalization and clearance kinetics within the cells, thus the phage is internalized within 15 minutes after administration and the amount of phage found in the cells increased with time. Phage particles were cleared from the cells after approximately one day. It was also found that the internalized system has no toxic effect on cells when applied in the media or once inside the cells. The phage intracellular pathway is shown to be via the endosomal pathway of integrin mediated endocytosis and that after 15 minutes the phage is located in the early endosomes, followed by relocation to the lysosomes within the next 2 hours.

## EXAMPLE 2

**Amyotrophic Lateral Sclerosis (ALS) Experiments**

**MATERIALS AND METHODS**

Amyotrophic Lateral Sclerosis (ALS) B6SJL-Tg (SOD1-G93A) 1Gur-J mice, which carry a high copy number of the mutated form of the SOD1 protein, which accelerates protein accumulation, disease onset and death were used. The average life-span of these mice is estimated to be 130-135 days, with disease onset, defined at 30% loss of motor performance (Urushitani et al. 2007), at 80-90 days.

Eight mice were divided into two groups. Four mice were intranasally administered with $10\mu l$ of live RGD-phages using a micropipette, and four mice were treated with 12 $\mu l$ of UV-inactivated RGD-phages in the same manner. The number of phages given to each mouse in each administration was $2.5 \times 10^{12}$. Mice were treated 2 times/week from age of 50 days for a period of 40 days (estimated motor decline onset), and 3 times/week from day 90 until they were sacrificed. Thirty four untreated mice were used as control to assess survival time.

Once a week, the mice were tested for motor strength using the Rotorod drum (SDI, California). Performance time on the Rotorod was monitored. Acceleration mode (rate 4) of up to 25RPM was set. Survival rate was assessed until mice could not reach food and water and/or when they could not roll over onto their stomach immediately after being placed on their side. Mice were sacrificed using $CO_2$ and their brain and spine were removed for fixation in PFA 4% for further analysis and histology.

UV inactivation of phage was conducted according to Delmastro et al. (1997). Phage solution was aliquoted in several 25 $\mu l$ drops, each placed in a well in a 24-well plate. The droplets were irradiated in 3 cycles (1 min pause between cycles) of irradiation with the U.V. Stratalinker, at 200,000 $\mu j$.

*Aggregation assay.*

ThT binding assay was exploited to assess the ability of UV-inactivated phage to dissolve amyloid aggregates (*in-vitro*) compared to ability of live phages to dissolve these aggregates. Amyloid-ß fibril formation was quantified by the Thioflavine-T (ThT) binding assay, which is specific to identifying amyloid fibrils. ThT associates rapidly with aggregated fibrils, giving rise to a new excitation (absorption) maximum at 435-450 nm and enhanced emission at 485 nm, as opposed to the 385 nm (excitation) and 445 nm emission of the free dye. This change is dependent on the aggregated state, as monomeric or dimeric peptides do not react, and guanidine dissociation of aggregates destroys the signal (LeVine H. 1993). Specimen emission was measured using a Perkin-Elmer model LS-50 spectrofluorimeter and indicated by arbitrary units.

Since this test is comparative (aggregation of amyloid-ß compared to aggregation of the peptide with phage), the absolute values are not as important as the relative aggregation percentages.

The aggregation percentage was calculated in the following way:

$$\% \text{ aggregation} = \frac{\text{Emission 482 nm (Amyloid } \beta \text{ incubated with phages)}}{\text{Emission 482 nm (Amyloid } \beta \text{ incubated alone)}} \times 100$$

Amyloid ß-aggregation was considered as 100%. The background values of phage and buffer solutions were always measured, and those values were omitted from the score of all results. In all the performed tests, the values for the phage solutions were almost like those of the buffer; therefore, it was concluded that phages do not form ThT-positive ß-sheet structures.

*Disaggregation Assay.*

AßP1-40 was incubated for 20 days at 37oC to promote aggregation. Phages or PBS were added and incubated with the preaggregated Aß for another 72 hr. The fluorescence was measured as described above.

**RESULTS**

Delay of onset of the disease, as well as an increase in survival rate, was observed in both groups of UV-inactivated RGD-phages and live RGD-phages, in comparison to the control untreated group. No observable adverse effects were seen in either group.

*Motor performance.*

Although both treated and untreated groups reached the same motor impairment end-point at the same age, Rotorod results shown in Figure 4 indicate that treated mice have better motor performance for a longer time than untreated mice, thus proving delay of disease onset.

*Survival rate.*

Longer life span was observed in both treated groups in comparison to the untreated group (Fig. 5).

*Histology results*

Histology results (Figs. 6A-6E) demonstrate phage penetration to neurons and astrocytes.

*UV-inactivation does not damage phage structure.*

In order to ensure that phages lose their infectious capability yet do not undergo structural modifications as a result of UV-inactivation, several experiments were conducted:

- Loss of phage infectivity of bacteria by irradiation.

- Electron Microscopy photos that enabled visualization of the filamentous structure of the phages.

- *In-vitro* disaggregating essay of amyloid-beta peptides

*Loss of phage infectivity of bacteria by irradiation.*

M13 filamentous phages, carrying the tetracycline resistance gene, were placed on 2YT petri dishes with added tetracycline. The plates were seeded with TG1 bacteria. TG1 cultures cannot grow on these plates without the phages infecting them and giving the bacteria the tetracycline resistance gene.
In Fig. 7, a control dish did not show any bacterial growth when PBS was placed on the seeded TG1 bacteria. When M13 phages were placed on the TG1 cultures, the bacteria were able to grow due to the tetracycline resistance conferred by the infecting phages. Different concentrations of phages (illustrated by the numbers on the culture dish) correlated with the number and density of TG1 cultures which were able to grow. When irradiated by UV, the phages lose their ability to infect the TG1 bacteria, thus preventing the bacteria from growing. It was found that one cycle of UV irradiation was enough for phage inactivation.

*EM visualization of UV-inactivated phage*

Electron microscopy images of UV-inactivated RGD-phages (Figs. 9A and 9B) were taken to ensure that the irradiation process did not damage the phage filamentous structure.

*Analysis of UV-inactivated phage interaction with amyloid aggregates using Thioflavin-T binding assay.*

This experiment was conducted with UV inactivated phages displaying the RGD peptide to demonstrate that UV inactivated phages not only maintain their structure but also retain their function and ability to disaggregate amyloid aggregates. Previous *in-vitro* experiments conducted in the lab of the present inventors have confirmed that live phages have the ability to disaggregate Aß aggregates.

Disaggregation test - 58μM Aβp 1-40 was incubated for 20 days. Filamentous phages were added and co-incubated for 72 hours. Amyloid content was measured using ThT.

A decline of 45% in AßP aggregation was observed when RGD-phages were added to preaggregated Aß. Same results were observed with RGD-phages that did not undergo the UV irradiation.

This experiment demonstrates that UV-irradiation does not damage RGD-phage ability to disaggregate amyloid aggregates. Moreover, this experiment shows that the RGD peptide displayed on the phages to allow their internalization into neurons does not damage the disaggregating ability of the phages (relevant to intracellular aggregates such as those found in Parkinson, Huntington and ALS).

## CONCLUSIONS

The evidence shown above demonstrates that filamentous phages have the ability to dissolve and disaggregate already formed intracellular amyloid plaques. The experiments in the mutant-SOD1-Tg mouse model of ALS disease show that SOD1 aggregates formed in the cytoplasm of cells in these mice can be dissolved and lead to improvement in mice health. Phage treatment of these mice not only improved their life expectancy, but also prolonged their motor ability and physical strength to a higher level relative to control mice.

The UV-inactivation experiments on the phages are another step towards developing a safe product for human use. UV-inactivated phages keep their filamentous structure undamaged and thus maintain their disaggregating ability of dissolving amyloid plaques.

The foregoing description of the specific embodiments will so fully reveal the general nature of the invention that others can, by applying knowledge within the skill of the art (including the contents of the references cited herein), readily modify and/or adapt for various applications such specific embodiments.

## <u>REFERENCES</u>

Banks and Kastin, Prog Brain Res., 91:139-4 (1992)

Barillari G, Gendelman R, Gallo RC, Ensoli B. 1993. The TAT protein of HIV-1, a growth factor for AIDS-Kaposi's sarcoma and cytokine-activated vascular cells, induces adhesion of the same cell-types by using integrin receptors recognizing the RGD amino-acid sequence. Proc. Natl. Acad. Sci. USA 90:7941-45.

Bruijn, L. I., M. K. Houseweart, et al. (1998). "Aggregation and motor neuron toxicity of an ALS-linked SOD1 mutant independent from wild-type SOD1." Science 281(5384):1851-4.

Buck, M. (1998) Trifluoroethanol and colleagues: cosolvents come of age. Recent studies with peptides and proteins. Q. Rev. Biophys. 31:297-355.

Carrera, M.R.A., Kaufmann, G.F., Mee, J.M., Meijler, M.M., Koob, G.F., Janda, K.D., "Treating cocaine addiction with viruses". Proc. Natl. Acad. Sci . USA, 101 (28) : 10416 (2004).

Caughey et al, "Secondary structure analysis of the scrapieassociated protein PrP 27-30 in water by infrared spectroscopy", Biochemistry 30:7672-7680 (1991)

Chartier Harlan et al, Nature 353:844 (1991)

Chiti, F., Taddei, N., Webster, P., Hamada, D., Fiaschi, T., Ramponi, G. & Dobson, C.M. (1999b) Acceleration of the folding of acylphosphatase by stabilisation of local secondary structure. Nat. Struct. Biol. 6:380-387.

Chiti, F., Webster, P., Taddei, N., Clark, A., Stefani, M., Ramponi, G. & Dobson, C.M. (1999a) Designing conditions for in vitro formation of amyloid protofilaments and fibrils. Proc. Natl Acad. Sci. USA 96:3590-3594.

Chou et al, Biopharm Drug Dispos. 18(4):335-46 (1997)

Chung, K.K., Zhang, Y., Lim, K.L., Tanaka, Y., Huang, H., Gao, J., Ross, C.A., Dawson, V.L. & Dawson, T.M. (2001) Parkin ubiquitinates the α-synuclein-interacting protein, synphilin-1: implications for Lewy-body formation in Parkinson disease. Nat. Med. 7:1144-1150.

Ciechanover, A. (2001) Linking ubiquitin, parkin and synphilin-1. Nat. Med. 7:1108-1109.

Conway, K.A., Harper, J.D. & Lansbury, P.T. (1998) Accelerated in vitro fibril formation by a mutant α-synuclein linked to early-onset Parkinson disease. Nat. Med. 4:1318-1320.

Cudkowicz, M., M. Qureshi, et al. (2004). "Measures and markers in amyotrophic lateral sclerosis." NeuroRx 1(2): 273-83.

De Gioia et al, "Conformational polymorphism of the amyloidogenic and neurotoxic peptide homologous to residues 106-126 of the prion protein", J Biol Chem 269:7859-7862 (1994)

Delmastro, P., A. Meola, et al. (1997). "Immunogenicity of filamentous phage displaying peptide mimotopes after oral administration." Vaccine 15(11): 1276-85.

Ding, T.T. & Harper, J.D. (1999) Analysis of amyloid-β assemblies using tapping mode atomic force microscopy under ambient conditions. Methods Enzymol. 309:510-525.

Dobson, C.M. (1999) Protein misfolding, evolution and disease. Trends Biochem. Sci. 24:329-332.

Draghia et al, Gene Therapy 2:418-423 (1995)

Elam, J. S., A. B. Taylor, et al. (2003). "Amyloid-like filaments and water-filled nanotubes formed by SOD1 mutant proteins linked to familial ALS." Nat Struct Biol 10(6): 461-7.

Ensoli B, Barillari G, Salahuddin SZ, Gallo RC, Wong-Staal F. 1990. Tat protein of HIV-1 stimulates growth of cells derived from Kaposi's sarcoma lesions of AIDs patients. Nature 345:84-86.

Fändrich, M., Fletcher, M.A. & Dobson, C.M. (2001) Amyloid fibrils from muscle myoglobin. Nature 410:165-166.

Ferreira, S.T. & De Felice, F.G. (2001) PABMB Lecture. Protein dynamics, folding and misfolding: from basic physical chemistry to human conformational diseases. FEBS Lett. 498:129-134.

Findeis, M.A. (2000) Approaches to discovery and characterization of inhibitors of amyloid ß-peptide polymerization. Biochim. Biophys. Acta 1502:76-84. Review.

Forloni et al,, "Neurotoxicity of a prion protein fragment", Nature 362:543-546 (1993)

Frenkel and Solomon, PNAS, 99:5675-5679 (2002)

Frenkel et al, "N-terminal EFRH sequence of Alzheimer's β-amyloid peptide represents the epitope of its anti-aggregating antibodies", J Neuroimmunology 88:85-90 (1998)

Gajdusek, Science 197:943-960 (1991)

Gardner JM, Hynes RO. 1985. Interaction of fibronectin with its receptor on platelets. Cell 42:439-48.

Gartner TK, Bennett JS. 1985. The tetrapeptide analog of the cell attachment site of fibronectin inhibits platelet aggregation and fibrinogen binding to activated platelets. J. Biol. Chem. 260:11891-94.

Goate et al, Nature 349:704 (1991)

Goedert, M., Spillantini, M.G. & Davies, S.W. (1998) Filamentous nerve cell inclusions in neurodegenerative diseases. Curr. Opin. Neurobiol. 8:619-632.

Gonzalez de Aguilar, J. L., A. Echaniz-Laguna, et al. (2007). "Amyotrophic lateral sclerosis: all roads lead to Rome." J Neurochem 101(5): 1153-60.

Greenwood, J., A. E. Willis and R. N. Perham (1991). "Multiple display of foreign peptides on a filamentous bacteriophage: Peptides from Plasmodium falciparum circumsporozoite protein as antigens." Journal of Molecular Biology 220(4):821-827.

Guijarro, J.I., Sunde, M., Jones, J.A., Campbell, I.D. & Dobson, C.M. (1998) Amyloid fibril formation by an SH3 domain. Proc. Natl Acad. Sci. USA 95:4224-4228.

Hardy, TINS, 20:154 (1997)

Harper, J.D., Wong, S.S., Lieber, C.M. & Lansbury, P.T. Jr (1999) Assembly of A βamyloid protofibrils: an in vitro model for a possible early event in Alzheimer's disease. Biochemistry 38:8972-8980.

Hart, S. L., A. M. Knight, et al. (1994). "Cell binding and internalization by filamentous phage displaying a cyclic Arg-Gly-Asp-containing peptide." J Biol Chem 269(17): 12468-74.

Hart, S. L., A. M. Knight, R. P. Harbottle, et al. (1994). "Cell binding and internalization by filamentous phage displaying a cyclic Arg-Gly-Asp-containing peptide." J Biol Chem 269 (17) :12468-74.

Horiuchi and Caughey, "Specific binding of normal prion protein to the scrapie form via a localized domain initiates its conversion to the protease-resistant state", EMBO J 18:3193-3203 (1999)

Ingram, D.K. (2001) Vaccine development for Alzheimer's disease: a shot of good news. Trends Neurosci. 24: 305-307.

Ivanenkov, V. V., F. Felici and A. G. Menon (1999b). "Targeted delivery of multivalent phage display vectors into mammalian cells." Biochim Biophys Acta 1448(3):463-72

Ivanenkov, V., F. Felici and A. G. Menon (1999a). "Uptake and intracellular fate of phage display vectors in mammalian cells." Biochim Biophys Acta 1448(3):450-62.

Jones, R. (2001) Blocking prion conversion. Highlights. Nat. Rev. Neurosci. 2:605.

Julien, J. P. and J. Kriz (2006). "Transgenic mouse models of amyotrophic lateral sclerosis." Biochim Biophys Acta 1762(11-12): 1013-24.

Kanyo et al, "Antibody binding defines a structure for an epitope that participates in the PrPC-->PrPSc conformational change", J Mol Biol. 293:855-863 (1999)

Koivunen E, Wang B, Ruoslahti E. 1995. Phage libraries displaying cyclic peptides with different ring size: ligand specificities of the RGD-directed integrins. Bio/Technology 13:265-70.

Korth, C., May, B.C., Cohen, F.E. & Prusiner, S.B. (2001) Acridine and phenothiazine derivatives as pharmacotherapeutics for prion disease. Proc. Natl Acad. Sci. USA 98:9836-9841.

Lee and Jameson (2005) Gene Therapy of pituitary diseases, J. Endocrinol. 185(3)353-362

LeVine H. (1993). "Thioflavine T interaction with synthetic Alzheimer's disease beta-amyloid peptides: detection of amyloid aggregation in solution." Protein Sci 2(3) :404-10.

Lomakin, A., Chung, D.S., Benedek, G.B., Kirschner, D.A. & Teplow, D.B. (1996) On the nucleation and growth of amyloid β-protein fibrils. detection of nuclei and quantitation of rate constants. Proc. Natl Acad. Sci. USA 93: 1125-1129.

Maggio JE, Mantyh PW, Brain amyloid-a physicochemical perspective. Brain Pathol. Apr;6(2):147-62 (1996)

Masliah, E. et al. (2000) Dopaminergic loss and inclusion body formation in α-synuclein mice; implications for neurodegenerative disorders. Science 287(5456), 1265-1269.

Mathison et al, J. Drug Target, 5(6):415-441 (1998)

Matsumoto, G., A. Stojanovic, et al. (2005). "Structural properties and neuronal toxicity of amyotrophic lateral sclerosis-associated Cu/Zn superoxide dismutase 1 aggregates." J Cell Biol 171(1):75-85.

Monaci P., et al., Curr Opin Mol Ther., 3(2):159-69 (2001)

Mountain (2000) Gene Therapy: the first decade, Trends Biotechnol. 18(3):119-128

Mullan et al, Nature Genet. 1:345 (1992)

Murrell et al, Science 254:97 (1991)

O'Neil, K. T., R. H. Hoess, S. A. Jackson, et al. (1992). "Identification of novel peptide antagonists for GPIIb/IIIa from a conformationally constrained phage peptide library." Proteins 14:509-515.

Pan et al, "Conversion of alpha-helices into beta-sheets features in the formation of the scrapie prion proteins", Proc Natl Acad Sci USA 90:10962-10966 (1993)

Peretz et al, "A conformational transition at the N terminus of the prion protein features in formation of the scrapie isoform", J Mol Biol 273:614-622 (1997)

Pierschbacher MD, Ruoslahti E. 1984a. The cell attachment activity of fibronectin can be duplicated by small fragments of the molecule. Nature 309:30-33

Pierschbacher MD, Ruoslahti E. 1984b. Variants of the cell recognition site of fibronectin that retain attachment-promoting activity. Proc. Natl. Acad. Sci. USA 81:5985-88

Plow EF, Pierschbacher MD, Ruoslahti E, Marguerie GA, Ginsberg MH. 1985. The effect of Arg-Gly-Asp containing peptides on fibrinogen and von Willebrand factor binding to platelets. Proc. Natl. Acad. Sci. USA 82:8057-61.

Raso, S.W. & King, J.K. (2000) Protein folding and human disease. In Mechanisms of Protein Folding (Pain R.H., ed.) Frontiers in Molecular Biology Series, pp. 406-428. Oxford University Press, Oxford.

Rochet, J.C. & Lansbury, P.T. Jr (2000) Amyloid fibrillogenesis: themes and variations. Curr. Opin. Struct. Biol. 10: 60-68.

Ruoslahti, E. (1996). RGD and other recognition sequences for integrins. Ann. Rev. Cell and Devel. Biol. 12:697-715.

Scherzinger, E., Lurz, R., Turmaine, M., Mangiarini, L., Hollenbach, B., Hasenbank, R., Bates, G.P., Davies, S.W., Lehrach, H. & Wanker, E.E. (1997) Huntingtin-encoded polyglutamine expansions form amyloid-like protein aggregates in vitro and in vivo. Cell 90:549-558.

Schlunegger, M.P., Bennett, M.J. & Eisenberg, D. (1997) Oligomer formation by 3D domain swapping: a model for protein assembly and misassembly. Adv. Protein Chem. 50:61-122.

Selvaggini et al, "Molecular characteristics of a protease-resistant, amyloidogenic and neurotoxic peptide homologous to residues 106-126 of the prion protein", Biochem Biophys Res Commun 194:1380-1386 (1993)

Serpell, L.C. (2000) Alzheimer's amyloid fibrils: structure and assembly. Biochim. Biophys. Acta 1502:16-30.

Silen and Agard, "The alpha-lytic protease pro-region does not require a physical linkage to activate the protease domain in vivo", Nature 341:462-464 (1989)

Soto, C. & Saborio, G.P. (2001) Prions: disease propagation and disease therapy by conformational transmission. Trends Mol. Med. 7:109-114.

Soto, C. (2001) Protein misfolding and disease; protein refolding and therapy. FEBS Lett. 498:204-207.

Spillantini, M.G., Schmidt, M.L., Lee, V.M., Trojanowski, J.Q., Jakes, R. & Goedert, M. (1997) α-Synuclein in Lewy bodies. Nature 388:839-840.

Stefanidakis and Koivunen, Peptide-mediated delivery of therapeutic and imaging agents into mammalian cells, Curr. Pharm. Des. (2004) 10(24):3033-3044

Suzuki S, Oldberg A, Hayman EG, Pierschbacher MD, Ruoslahti E. 1985. Complete amino acid sequence of human vitronectin deduced from cDNA. Similarity of cell attachment sites in vitronectin and fibronectin. EMBO J. 4:2519-24.

Tagliavini et al, "Synthetic peptides homologous to prion protein residues 106-147 form amyloid-like fibrils in vitro", Proc Natl Acad Sci USA 90:9678-9682 (1993)

Taylor, J. P., J. Hardy, et al. (2002). "Toxic proteins in neurodegenerative disease." Science 296(5575): 1991-5.

Turmaine, M., Raza, A., Mahal, A., Mangiarini, L., Bates, G.P. & Davies, S.W. (2000) Nonapoptotic neurodegeneration in a transgenic mouse model of Huntington's disease. Proc. Natl Acad. Sci. USA 97:8093-8097.

Urushitani, M., S. A. Ezzi, et al. (2007). "Therapeutic effects of immunization with mutant superoxide dismutase in mice models of amyotrophic lateral sclerosis." Proc Natl Acad Sci U S A 104(7):2495-500.

Uversky, V.N., Li, J. & Fink, A.L. (2001) Evidence for a partially folded intermediate in α-synuclein fibril formation. J. Biol. Chem. 276:10737-10744.

Vogel BE, Lee S-J, Hildebrand A, Craig W, Pierschbacher M, et al. 1993. A novel integrin specificity exemplified by binding of the αvβ5 integrin to the basic domain of the HIV Tat protein and vitronectin. J. Cell Biol. 121:461-68.

Walsh, D.M., Hartley, D.M., Kusumoto, Y., Fezoui, Y., Condron, M.M., Lomakin, A., Benedek, G.B., Selkoe, D.J. & Teplow, D.B. (1999) Amyloid β-protein fibrillogenesis. Structure and biological activity of protofibrillar intermediates. J. Biol. Chem. 274:25945-25952.

Watanabe, M., M. Dykes-Hoberg, et al. (2001). "Histological evidence of protein aggregation in mutant SOD1 transgenic mice and in amyotrophic lateral sclerosis neural tissues." Neurobiol Dis 8(6): 933-41.

Weeks BS, Desal K, Lowenstein PM, Klotman ME, Klotman PE, et al. 1993. Identification of a novel cell attachment domain in the HIV-1 Tat protein and its 90-kDa cell surface binding protein. J. Biol. Chem. 268:5279-84.

Wilesmith and Wells, Curr Top Microbiol Immunol 172:21-38 (1991)

Wong, P. C., H. Cai, et al. (2002). "Genetically engineered mouse models of neurodegenerative diseases." Nat Neurosci 5(7): 633-9.

Wood, J. D., T. P. Beaujeux, et al. (2003). "Protein aggregation in motor neurone disorders." Neuropathol Appl Neurobiol 29(6):529-45.

Yamada KM, Kennedy DW. 1984. Dualistic nature of adhesive protein function: Fibronectin and its biologically active peptide fragments can autoinhibit fibronectin function. J. Cell Biol. 99:29-36

Young AA. et al, FEBS Lett, 343(3):237-41 (1994)

**Claims**

1. Filamentous bacteriophage, which carries a peptide sequence comprising a mammalian cell adhesion sequence, wherein the mammalian cell adhesion sequence is displayed so as to be capable of causing internalization of said filamentous bacteriophage into cells, for use in inhibiting or treating a disease associated with intracellular formation of protein fibrillar inclusions or aggregates, the disease being a neurodegenerative disease or system amyloidosis.

2. Filamentous bacteriophage for use according to claim 1, which does not display an antibody or a non-filamentous bacteriophage antigen other than said peptide sequence.

3. Filamentous bacteriophage for use according to claim 2, being selected from the group consisting of M13, f1, and fd bacteriophages, and mixtures thereof, the filamentous bacteriphage preferably being M13.

4. Filamentous bacteriophage for use according to claim 2 or 3, wherein about 150 copies of said peptide sequence is displayed on the surface of said filamentous bacteriophage.

5. Filamentous bacteriophage for use according to any of the preceding claims, wherein said mammalian cell adhesion sequence is an Arg-Gly-Asp (RGD) cell adhesion sequence.

6. Filamentous bacteriophage for use according to claim 5, wherein said peptide sequence comprising said RGD cell adhesion sequence is cyclic.

7. Filamentous bacteriophage for use according to claim 6, wherein said peptide sequence comprises said RGD cell adhesion sequence of SEQ ID NO:1.

8. Filamentous bacteriophage for use according to claim 6, wherein said peptide sequence comprises said RGD cell adhesion sequence of SEQ ID NO:2.

9. Filamentous bacteriophage for use according to any of the preceding claims, wherein the disease is selected from the group consisting of Alzheimer's disease, dementia with Lewy bodies, Parkinson's disease, and amyotrophic lateral sclerosis.

10. Filamentous bacteriophage for use according to any of the claims 2 - 9, wherein the filamentous bacteriophage is a UV-inactivated filamentous bacteriophage that maintains its filamentous structure.

11. Filamentous bacteriophage for use according to any of the claims 2 - 10, wherein an effective amount of the filamentous bacteriophage is administered intranasally.

12. Pharmaceutical composition for use in inhibiting or treating a disease associated with intracellular formation of protein fibrillar inclusions or aggregates, the disease being a neurodegenerative disease or system amyloidosis, comprising a pharmaceutically acceptable carrier or excipient and, as an active ingredient, an effective amount of a filamentous bacteriophage as defined in any of the claims 2-11.

13. Pharmaceutical composition for use according to claim 12, wherein an effective amount of said filamentous bacteriophage is administered intranasally.

**Patentansprüche**

1. Fadenförmiger Bakteriophage, welcher eine Peptidsequenz umfassend eine Säugerzellen-Adhäsionssequenz, wobei die Säugerzellen-Adhäsionssequenz so dargestellt ist, dass diese in der Lage ist, eine Internalisierung des fadenförmigen Bakteriophagen in Zellen zu veranlassen, zur Verwendung beim Hemmen oder Behandeln einer mit einer intrazellularen Bildung von fibrilären Eiweißeinschlüssen oder -aggregaten verbundene Erkrankung, wobei die Erkrankung eine neurodegenerative Erkrankung oder systemische Amyloidose ist.

2. Fadenförmiger Bakteriophage zur Verwendung nach Anspruch 1, welcher keinen Antikörper oder kein nicht fadenförmiges Bakteriophagen-Antigen außer dieser Peptidsequenz darstellt.

3. Fadenförmiger Bakteriophage zur Verwendung nach Anspruch 2, ausgewählt aus der Gruppe bestehend aus M13-, f1- und fd-Bakteriophagen und Mischungen davon, wobei der fadenförmige Bakteriophage vorzugsweise M13 ist.

4. Fadenförmiger Bakteriophage zur Verwendung nach Anspruch 2 oder 3, in welchem etwa 150 Kopien der Peptidsequenz auf der Oberfläche des fadenförmigen Bakteriophagen dargestellt sind.

5. Fadenförmiger Bakteriophage zur Verwendung nach einem der vorhergehenden Ansprüche, in welchem die Säugerzellen-Adhäsionssequenz eine Arg-Gly-Asp (RGD) Zellenadhäsionssequenz ist.

6. Fadenförmiger Bakteriophage zur Verwendung nach Anspruch 5, in welchem die Peptidsequenz umfassend die RGD Zellen-Adhäsionssequenz zyklisch ist.

7. Fadenförmiger Bakteriophage zur Verwendung nach Anspruch 6, in welchem die Peptidsequenz die RGD Zellen-Adhäsionssequenz SEQ ID NO:1 umfasst.

8. Fadenförmiger Bakteriophage zur Verwendung nach Anspruch 6, in welchem die Peptidsequenz die RGD Zellen-Adhäsionssequenz SEQ ID NO:2 umfasst.

9. Fadenförmiger Bakteriophage zur Verwendung einem der vorhergehenden Ansprüche, in welchem die Erkrankung ausgewählt ist aus der Gruppe bestehend aus Alzheimererkrankung, Demenz mit Lewy-Körperchen, Parkinsonerkrankung und amyotrophe laterale Sklerose.

10. Fadenförmiger Bakteriophage zur Verwendung nach einem der Ansprüche 2 bis 9, in welchem der fadenförmige Bakteriophage ein UV-inaktivierter fadenförmiger Bakteriophage ist, der seine fadenförmige Struktur behält.

11. Fadenförmiger Bakteriophage zur Verwendung nach einem der Ansprüche 2 bis 10, in welchem eine effektive Menge des fadenförmigen Bakteriophagen intranasal verabreicht wird.

12. Pharmazeutische Zusammensetzung zum Verwenden beim Hemmen oder Behandeln einer mit einer intrazellularen Bildung von fibrilären Einschlüssen oder Aggregaten aus Eiweiß verbundener Erkrankung, wobei die Erkrankung eine neurodegenerative Erkrankung oder eine systemische Amyloidose ist, mit einem pharmazeutisch akzeptablen Träger oder Hilfsstoff und, als ein aktiver Inhaltsstoff, einer effektiven Menge eines fadenförmigen Bakteriophagen nach einem der Ansprüche 2 bis 11.

**13.** Pharmazeutische Zusammensetzung zur Verwendung nach Anspruch 12, in welcher eine effektive Menge des fadenförmigen Bakteriophagen internasal verabreicht wird.

**Revendications**

**1.** Bactériophage filamenteux, qui porte une séquence peptidique comprenant une séquence d'adhésion à des cellules de mammifère, dans lequel la séquence d'adhésion à des cellules de mammifère est exposée de manière à être capable de provoquer l'internalisation dudit bactériophage filamenteux dans des cellules, pour une utilisation dans l'inhibition ou le traitement d'une maladie associée à la formation intracellulaire d'inclusions ou d'agrégats fibrillaires de protéines, la maladie étant une maladie neurodégénérative ou l'amyloïdose d'un système.

**2.** Bactériophage filamenteux pour une utilisation suivant la revendication 1, qui n'expose pas d'anticorps ou d'antigène de bactériophage non filamenteux autre que ladite séquence peptidique.

**3.** Bactériophage filamenteux pour une utilisation suivant la revendication 2, qui est choisi dans le groupe consistant en les bactériophages M13, f1 et fd, et leurs mélanges, le bactériophage filamenteux étant de préférence le bactériophage M13.

**4.** Bactériophage filamenteux pour une utilisation suivant la revendication 2 ou 3, dans lequel environ 150 copies de ladite séquence peptidique sont exposées sur la surface dudit bactériophage filamenteux.

**5.** Bactériophage filamenteux pour une utilisation suivant l'une quelconque des revendications précédentes, dans lequel ladite séquence d'adhésion à des cellules de mammifère est une séquence d'adhésion à des cellules Arg-Gly-Asp (RGD).

**6.** Bactériophage filamenteux pour une utilisation suivant la revendication 5, dans lequel ladite séquence peptidique comprenant ladite séquence d'adhésion à des cellules RGD est cyclique.

**7.** Bactériophage filamenteux pour une utilisation suivant la revendication 6, dans lequel ladite séquence peptidique comprend ladite séquence d'adhésion à des cellules RGD de la SEQ ID NO:1.

**8.** Bactériophage filamenteux pour une utilisation suivant la revendication 6, dans lequel ladite séquence peptidique comprend ladite séquence d'adhésion à des cellules RGD de la SEQ ID NO:2.

**9.** Bactériophage filamenteux pour une utilisation suivant l'une quelconque des revendications précédentes, la maladie étant choisie dans le groupe consistant en la maladie d'Alzheimer, la démence avec présence de corps de Lewy, la maladie de Parkinson et la sclérose latérale amyotrophique.

**10.** Bactériophage filamenteux pour une utilisation suivant l'une quelconque des revendications 2 à 9, ledit bactériophage filamenteux étant un bactériophage filamenteux inactivé par UV qui conserve sa structure filamenteuse.

**11.** Bactériophage filamenteux pour une utilisation suivant l'une quelconque des revendications 2 à 10, une quantité efficace du bactériophage filamenteux étant administrée par voie intranasale.

**12.** Composition pharmaceutique pour une utilisation dans l'inhibtion ou le traitement d'une maladie associée à la formation intracellulaire d'inclusions ou d'agrégrats fibrillaires de protéines, la maladie étant une maladie neurodégénérative ou l'amyloïdose d'un système, comprenant un support pharmaceutiquement ou excipient pharmaceutiquement acceptable et, comme ingrédient actif, une quantité efficace d'un bactériophage filamenteux tel que défini dans l'une quelconque des revendications 2 à 11.

**13.** Composition pharmaceutique pour une utilisation suivant la revendication 12, dans laquelle une quantité efficace dudit bactériophage filamenteux est administrée par voie intranasale.

Figure 1A

Figure 1B

Figure 1C

Figure 2A

Figure 2B

Figure 2C

Figure 3A

Figure 3B

Figure 4

Figure 5

Figure 6A                    Figure 6B                    Figure 6C

Figure 6D

Figure 6E

Control - TG1 -    TG1 infected with

Figure 7

Figure 8

Figure 9A

Figure 9B

Figure 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 60832229 B

### Non-patent literature cited in the description

- **Hardy.** *TINS,* 1997, vol. 20, 154
- Remington's Pharmaceutical Sciences. Mack Publishing Co,
- **Fingl et al.** The Pharmacological Basis of Therapeutics. 1975, 1
- **Banks ; Kastin.** *Prog Brain Res.,* 1992, vol. 91, 139-4
- **Barillari G ; Gendelman R ; Gallo RC ; Ensoli B.** The TAT protein of HIV-1, a growth factor for AIDS-Kaposi's sarcoma and cytokine-activated vascular cells, induces adhesion of the same cell-types by using integrin receptors recognizing the RGD amino-acid sequence. *Proc. Natl. Acad. Sci. USA,* 1993, vol. 90, 7941-45
- **Bruijn, L. I. ; M. K. Houseweart et al.** Aggregation and motor neuron toxicity of an ALS-linked SOD1 mutant independent from wild-type SOD1. *Science,* 1998, vol. 281 (5384), 1851-4
- **Buck, M.** Trifluoroethanol and colleagues: cosolvents come of age. Recent studies with peptides and proteins. *Q. Rev. Biophys.,* 1998, vol. 31, 297-355
- **Carrera, M.R.A. ; Kaufmann, G.F. ; Mee, J.M. ; Meijler, M.M. ; Koob, G.F. ; Janda, K.D.** Treating cocaine addiction with viruses. *Proc. Natl. Acad. Sci . USA,* 2004, vol. 101 (28), 10416
- **Caughey et al.** Secondary structure analysis of the scrapieassociated protein PrP 27-30 in water by infrared spectroscopy. *Biochemistry,* 1991, vol. 30, 7672-7680
- **Chartier Harlan et al.** *Nature,* 1991, vol. 353, 844
- **Chiti, F. ; Webster, P. ; Hamada, D. ; Fiaschi, T. ; Ramponi, G. ; Dobson, C.M.** Acceleration of the folding of acylphosphatase by stabilisation of local secondary structure. *Nat. Struct. Biol.,* 1999, vol. 6, 380-387
- **Chiti, F. ; Webster, P. ; Taddei, N. ; Clark, A. ; Stefani, M. ; Ramponi, G. ; Dobson, C.M.** Designing conditions for in vitro formation of amyloid protofilaments and fibrils. *Proc. Natl Acad. Sci. USA,* 1999, vol. 96, 3590-3594
- **Chou et al.** *Biopharm Drug Dispos.,* 1997, vol. 18 (4), 335-46

- **Chung, K.K. ; Zhang, Y. ; Lim, K.L. ; Tanaka, Y. ; Huang, H. ; Gao, J. ; Ross, C.A. ; Dawson, V.L. ; Dawson, T.M.** Parkin ubiquitinates the α-synuclein-interacting protein, synphilin-1: implications for Lewy-body formation in Parkinson disease. *Nat. Med.,* 2001, vol. 7, 1144-1150
- **Ciechanover, A.** Linking ubiquitin, parkin and synphilin-1. *Nat. Med.,* 2001, vol. 7, 1108-1109
- **Conway, K.A. ; Harper, J.D. ; Lansbury, P.T.** Accelerated in vitro fibril formation by a mutant α-synuclein linked to early-onset Parkinson disease. *Nat. Med.,* 1998, vol. 4, 1318-1320
- **Cudkowicz, M. ; M. Qureshi et al.** Measures and markers in amyotrophic lateral sclerosis. *NeuroRx,* 2004, vol. 1 (2), 273-83
- **De Gioia et al.** Conformational polymorphism of the amyloidogenic and neurotoxic peptide homologous to residues 106-126 of the prion protein. *J Biol Chem,* 1994, vol. 269, 7859-7862
- **Delmastro, P. ; A. Meola et al.** Immunogenicity of filamentous phage displaying peptide mimotopes after oral administration. *Vaccine,* 1997, vol. 15 (11), 1276-85
- **Ding, T.T. ; Harper, J.D.** Analysis of amyloid-β assemblies using tapping mode atomic force microscopy under ambient conditions. *Methods Enzymol.,* 1999, vol. 309, 510-525
- **Dobson, C.M.** Protein misfolding, evolution and disease. *Trends Biochem.,* 1999, vol. 24, 329-332
- **Draghia et al.** *Gene Therapy,* 1995, vol. 2, 418-423
- **Elam, J. S. ; A. B. Taylor et al.** Amyloid-like filaments and water-filled nanotubes formed by SOD1 mutant proteins linked to familial ALS. *Nat Struct Biol,* 2003, vol. 10 (6), 461-7
- **Ensoli B ; Barillari G ; Salahuddin SZ ; Gallo RC ; Wong-Staal F.** Tat protein of HIV-1 stimulates growth of cells derived from Kaposi's sarcoma lesions of AIDs patients. *Nature,* 1990, vol. 345, 84-86
- **Fändrich, M. ; Fletcher, M.A. ; Dobson, C.M.** Amyloid fibrils from muscle myoglobin. *Nature,* 2001, vol. 410, 165-166

- **Ferreira, S.T. ; De Felice, F.G.** PABMB Lecture. Protein dynamics, folding and misfolding: from basic physical chemistry to human conformational diseases. *FEBS Lett.,* 2001, vol. 498, 129-134
- **Findeis, M.A.** Approaches to discovery and characterization of inhibitors of amyloid ß-peptide polymerization. *Biochim. Biophys. Acta,* 2000, vol. 1502, 76-84
- **Forloni et al.** Neurotoxicity of a prion protein fragment. *Nature,* 1993, vol. 362, 543-546
- **Frenkel ; Solomon.** *PNAS,* 2002, vol. 99, 5675-5679
- **Frenkel et al.** N-terminal EFRH sequence of Alzheimer's β-amyloid peptide represents the epitope of its anti-aggregating antibodies. *J Neuroimmunology,* 1998, vol. 88, 85-90
- **Gajdusek.** *Science,* 1991, vol. 197, 943-960
- **Gardner JM ; Hynes RO.** Interaction of fibronectin with its receptor on platelets. *Cell,* 1985, vol. 42, 439-48
- **Gartner TK ; Bennett JS.** The tetrapeptide analog of the cell attachment site of fibronectin inhibits platelet aggregation and fibrinogen binding to activated platelets. *J. Biol. Chem.,* 1985, vol. 260, 11891-94
- **Goate et al.** *Nature,* 1991, vol. 349, 704
- **Goedert, M. ; Spillantini, M.G. ; Davies, S.W.** Filamentous nerve cell inclusions in neurodegenerative diseases. *Curr. Opin. Neurobiol.,* 1998, vol. 8, 619-632
- **Gonzalez de Aguilar, J. L. ; A. Echaniz-Laguna et al.** Amyotrophic lateral sclerosis: all roads lead to Rome. *J Neurochem,* 2007, vol. 101 (5), 1153-60
- **Greenwood, J. ; A. E. Willis ; R. N. Perham.** Multiple display of foreign peptides on a filamentous bacteriophage: Peptides from Plasmodium falciparum circumsporozoite protein as antigens. *Journal of Molecular Biology,* 1991, vol. 220 (4), 821-827
- **Guijarro, J.I. ; Sunde, M. ; Jones, J.A. ; Campbell, I.D. ; Dobson, C.M.** Amyloid fibril formation by an SH3 domain. *Proc. Natl Acad. Sci. USA,* 1998, vol. 95, 4224-4228
- **Harper, J.D. ; Wong, S.S. ; Lieber, C.M. ; Lansbury, P.T. Jr.** Assembly of A βamyloid protofibrils: an in vitro model for a possible early event in Alzheimer's disease. *Biochemistry,* 1999, vol. 38, 8972-8980
- **Hart, S. L. ; A. M. Knight et al.** Cell binding and internalization by filamentous phage displaying a cyclic Arg-Gly-Asp-containing peptide. *J Biol Chem,* 1994, vol. 269 (17), 12468-74
- **Hart, S. L. ; A. M. Knight ; R. P. Harbottle et al.** Cell binding and internalization by filamentous phage displaying a cyclic Arg-Gly-Asp-containing peptide. *J Biol Chem,* 1994, vol. 269 (17), 12468-74
- **Horiuchi ; Caughey.** Specific binding of normal prion protein to the scrapie form via a localized domain initiates its conversion to the protease-resistant state. *EMBO J,* 1999, vol. 18, 3193-3203
- **Ingram, D.K.** Vaccine development for Alzheimer's disease: a shot of good news. *Trends Neurosci.,* 2001, vol. 24, 305-307
- **Ivanenkov, V. V. ; F. Felici ; A. G. Menon.** Targeted delivery of multivalent phage display vectors into mammalian cells. *Biochim Biophys Acta,* 1999, vol. 1448 (3), 463-72
- **Ivanenkov, V. ; F. Felici ; A. G. Menon.** Uptake and intracellular fate of phage display vectors in mammalian cells. *Biochim Biophys Acta,* 1999, vol. 1448 (3), 450-62
- **Jones, R.** Blocking prion conversion. *Highlights. Nat. Rev. Neurosci.,* 2001, vol. 2, 605
- **Julien, J. P. ; J. Kriz.** Transgenic mouse models of amyotrophic lateral sclerosis. *Biochim Biophys Acta,* 2006, vol. 1762 (11-12), 1013-24
- **Kanyo et al.** Antibody binding defines a structure for an epitope that participates in the PrPC-->PrPSc conformational change. *J Mol Biol.,* 1999, vol. 293, 855-863
- **Koivunen E ; Wang B ; Ruoslahti E.** Phage libraries displaying cyclic peptides with different ring size: ligand specificities of the RGD-directed integrins. *Bio/Technology,* 1995, vol. 13, 265-70
- **Korth, C. ; May, B.C. ; Cohen, F.E. ; Prusiner, S.B.** Acridine and phenothiazine derivatives as pharmacotherapeutics for prion disease. *Proc. Natl Acad. Sci. USA,* 2001, vol. 98, 9836-9841
- **Lee ; Jameson.** Gene Therapy of pituitary diseases. *J. Endocrinol.,* 2005, vol. 185 (3), 353-362
- **LeVine H.** Thioflavine T interaction with synthetic Alzheimer's disease beta-amyloid peptides: detection of amyloid aggregation in solution. *Protein Sci,* 1993, vol. 2 (3), 404-10
- **Lomakin, A. ; Chung, D.S. ; Benedek, G.B. ; Kirschner, D.A. ; Teplow, D.B.** On the nucleation and growth of amyloid β-protein fibrils. detection of nuclei and quantitation of rate constants. *Proc. Natl Acad. Sci. USA,* 1996, vol. 93, 1125-1129
- **Maggio JE ; Mantyh PW.** Brain amyloid-a physicochemical perspective. *Brain Pathol.,* April 1996, vol. 6 (2), 147-62
- **Masliah, E. et al.** Dopaminergic loss and inclusion body formation in α-synuclein mice; implications for neurodegenerative disorders. *Science,* 2000, vol. 287 (5456), 1265-1269
- **Mathison et al.** *J. Drug Target,* 1998, vol. 5 (6), 415-441
- **Matsumoto, G. ; A. Stojanovic et al.** Structural properties and neuronal toxicity of amyotrophic lateral sclerosis-associated Cu/Zn superoxide dismutase 1 aggregates. *J Cell Biol,* 2005, vol. 171 (1), 75-85

- **Monaci P. et al.** *Curr Opin Mol Ther.,* 2001, vol. 3 (2), 159-69
- **Mountain.** Gene Therapy: the first decade. *Trends Biotechnol.,* 2000, vol. 18 (3), 119-128
- **Mullan et al.** *Nature Genet.,* 1992, vol. 1, 345
- **Murrell et al.** *Science,* 1991, vol. 254, 97
- **O'Neil, K. T. ; R. H. Hoess ; S. A. Jackson et al.** Identification of novel peptide antagonists for GPIIb/IIIa from a conformationally constrained phage peptide library. *Proteins,* 1992, vol. 14, 509-515
- **Pan et al.** Conversion of alpha-helices into beta-sheets features in the formation of the scrapie prion proteins. *Proc Natl Acad Sci USA,* 1993, vol. 90, 10962-10966
- **Peretz et al.** A conformational transition at the N terminus of the prion protein features in formation of the scrapie isoform. *J Mol Biol,* 1997, vol. 273, 614-622
- **Pierschbacher MD ; Ruoslahti E.** The cell attachment activity of fibronectin can be duplicated by small fragments of the molecule. *Nature,* 1984, vol. 309, 30-33
- **Pierschbacher MD ; Ruoslahti E.** Variants of the cell recognition site of fibronectin that retain attachment- promoting activity. *Proc. Natl. Acad. Sci. USA,* 1984, vol. 81, 5985-88
- **Plow EF ; Pierschbacher MD ; Ruoslahti E ; Marguerie GA ; Ginsberg MH.** The effect of Arg-Gly-Asp containing peptides on fibrinogen and von Willebrand factor binding to platelets. *Proc. Natl. Acad. Sci. USA,* 1985, vol. 82, 8057-61
- Protein folding and human disease. In Mechanisms of Protein Folding. **Raso, S.W. ; King, J.K.** Frontiers in Molecular Biology Series. Oxford University Press, 2000, 406-428
- **Rochet, J.C. ; Lansbury, P.T. Jr.** Amyloid fibrillogenesis: themes and variations. *Curr. Opin. Struct. Biol.,* 2000, vol. 10, 60-68
- **Ruoslahti, E.** RGD and other recognition sequences for integrins. *Ann. Rev. Cell and Devel. Biol.,* 1996, vol. 12, 697-715
- **Scherzinger, E. ; Lurz, R. ; Turmaine, M. ; Mangiarini, L. ; Hollenbach, B. ; Hasenbank, R. ; Bates, G.P. ; Davies, S.W. ; Lehrach, H. ; Wanker, E.E.** Huntingtin-encoded polyglutamine expansions form amyloid-like protein aggregates in vitro and in vivo. *Cell,* 1997, vol. 90, 549-558
- **Schlunegger, M.P. ; Bennett, M.J. ; Eisenberg, D.** Oligomer formation by 3D domain swapping: a model for protein assembly and misassembly. *Adv. Protein Chem.,* 1997, vol. 50, 61-122
- **Selvaggini et al.** Molecular characteristics of a protease-resistant, amyloidogenic and neurotoxic peptide homologous to residues 106-126 of the prion protein. *Biochem Biophys Res Commun,* 1993, vol. 194, 1380-1386
- **Serpell, L.C.** Alzheimer's amyloid fibrils: structure and assembly. *Biochim. Biophys. Acta,* 2000, vol. 1502, 16-30
- **Silen ; Agard.** The alpha-lytic protease pro-region does not require a physical linkage to activate the protease domain in vivo. *Nature,* 1989, vol. 341, 462-464
- **Soto, C. ; Saborio, G.P.** Prions: disease propagation and disease therapy by conformational transmission. *Trends Mol. Med.,* 2001, vol. 7, 109-114
- **Soto, C.** Protein misfolding and disease; protein refolding and therapy. *FEBS Lett.,* 2001, vol. 498, 204-207
- **Spillantini, M.G. ; Schmidt, M.L. ; Lee, V.M. ; Trojanowski, J.Q. ; Jakes, R. ; Goedert, M.** α-Synuclein in Lewy bodies. *Nature,* 1997, vol. 388, 839-840
- **Stefanidakis ; Koivunen.** Peptide-mediated delivery of therapeutic and imaging agents into mammalian cells. *Curr. Pharm. Des.,* 2004, vol. 10 (24), 3033-3044
- **Suzuki S ; Oldberg A ; Hayman EG ; Pierschbacher MD ; Ruoslahti E.** Complete amino acid sequence of human vitronectin deduced from cDNA. Similarity of cell attachment sites in vitronectin and fibronectin. *EMBO J.,* 1985, vol. 4, 2519-24
- **Tagliavini et al.** Synthetic peptides homologous to prion protein residues 106-147 form amyloid-like fibrils in vitro. *Proc Natl Acad Sci USA,* 1993, vol. 90, 9678-9682
- **Taylor, J. P. ; J. Hardy et al.** Toxic proteins in neurodegenerative disease. *Science,* 2002, vol. 296 (5575), 1991-5
- **Turmaine, M. ; Raza, A. ; Mahal, A. ; Mangiarini, L. ; Bates, G.P. ; Davies, S.W.** Nonapoptotic neurodegeneration in a transgenic mouse model of Huntington's disease. *Proc. Natl Acad. Sci. USA,* 2000, vol. 97, 8093-8097
- **Urushitani, M. ; S. A. Ezzi et al.** Therapeutic effects of immunization with mutant superoxide dismutase in mice models of amyotrophic lateral sclerosis. *Proc Natl Acad Sci U S A,* 2007, vol. 104 (7), 2495-500
- **Uversky, V.N. ; Li, J. ; Fink, A.L.** Evidence for a partially folded intermediate in α-synuclein fibril formation. *J. Biol. Chem.,* 2001, vol. 276, 10737-10744
- **Vogel BE ; Lee S-J ; Hildebrand A ; Craig W ; Pierschbacher M et al.** A novel integrin specificity exemplified by binding of the αvβ5 integrin to the basic domain of the HIV Tat protein and vitronectin. *J. Cell Biol.,* 1993, vol. 121, 461-68
- **Walsh, D.M. ; Hartley, D.M. ; Kusumoto, Y. ; Fezoui, Y. ; Condron, M.M. ; Lomakin, A. ; Benedek, G.B. ; Selkoe, D.J. ; Teplow, D.B.** Amyloid β-protein fibrillogenesis. Structure and biological activity of protofibrillar intermediates. *J. Biol. Chem.,* 1999, vol. 274, 25945-25952

- **Watanabe, M. ; M. Dykes-Hoberg et al.** Histological evidence of protein aggregation in mutant SOD1 transgenic mice and in amyotrophic lateral sclerosis neural tissues. *Neurobiol Dis,* 2001, vol. 8 (6), 933-41
- **Weeks BS ; Desal K ; Lowenstein PM ; Klotman ME ; Klotman PE et al.** Identification of a novel cell attachment domain in the HIV-1 Tat protein and its 90-kDa cell surface binding protein. *J. Biol. Chem.,* 1993, vol. 268, 5279-84
- **Wilesmith ; Wells.** *Curr Top Microbiol Immunol,* 1991, vol. 172, 21-38

- **Wong, P. C. ; H. Cai et al.** Genetically engineered mouse models of neurodegenerative diseases. *Nat Neurosci,* 2002, vol. 5 (7), 633-9
- **Wood, J. D. ; T. P. Beaujeux et al.** Protein aggregation in motor neurone disorders. *Neuropathol Appl Neurobiol,* 2003, vol. 29 (6), 529-45
- **Yamada KM ; Kennedy DW.** Dualistic nature of adhesive protein function: Fibronectin and its biologically active peptide fragments can autoinhibit fibronectin function. *J. Cell Biol.,* 1984, vol. 99, 29-36
- **Young AA. et al.** *FEBS Lett,* 1994, vol. 343 (3), 237-41